(19) Europäisches Patentamt
European Patent Office
Office européen des brevets

(11) EP 4 591 890 A1

(12) EUROPEAN PATENT APPLICATION

(43) Date of publication:
**30.07.2025 Bulletin 2025/31**

(21) Application number: **25153528.2**

(22) Date of filing: **23.01.2025**

(51) International Patent Classification (IPC):
***A61K 51/12*** *(2006.01)* ***A61K 9/51*** *(2006.01)*
***A61P 35/00*** *(2006.01)*

(52) Cooperative Patent Classification (CPC):
**A61K 51/1244; A61K 9/5123; A61K 51/1234; A61K 51/1251**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.01.2024 JP 2024008841**
**08.11.2024 JP 2024195650**

(71) Applicant: **Kabushiki Kaisha Toshiba**
**Tokyo 105-0023 (JP)**

(72) Inventors:
- **OKUMURA, Shu**
**Minato-ku, Tokyo (JP)**
- **ISHIHARA, Mitsuko**
**Minato-ku, Tokyo (JP)**
- **SUZUKI, Yasuhiro**
**Kawasaki-shi, Kanagawa (JP)**
- **KATO, Masayo**
**Kawasaki-shi, Kanagawa (JP)**
- **TEZUKA, Masaru**
**Minato-ku, Tokyo, 105-8001 (JP)**

(74) Representative: **Hoffmann Eitle**
**Patent- und Rechtsanwälte PartmbB**
**Arabellastraße 30**
**81925 München (DE)**

# (54) LIPID NANOPARTICLE FOR RADIATION THERAPY, MANUFACTURING METHOD, COMBINATION COMPOSITION, AND KIT

(57) According to one arrangement, a radiotherapy lipid nanoparticle (10) includes a biodegradable lipid nanoparticle (11), and a radioactive material (12) as an active ingredient. The radioactive material is bound to the biodegradable lipid nanoparticle and located outside of the biodegradable lipid nanoparticle.

EP 4 591 890 A1

## Description

FIELD

The present disclosure relates to a lipid nanoparticle for radiation therapy, manufacturing method, combination composition, and kit.

BACKGROUND

Radioactive materials are widely used in therapy and diagnosis. For example, targeted isotope therapy is a method of treating target cells using radioactive materials. Conventionally, artificial radioactive materials, i.e., artificial radionuclides, were commonly produced by nuclear reactors such as research reactors. In recent years, radioactive materials produced by research reactors have been in short supply worldwide, and treatments using radioactive materials produced by accelerators have been attracting attention instead.

BRIEF DESCRIPTION OF THE DRAWINGS

FIG. 1 is a schematic diagram showing one example of a radiotherapy lipid nanoparticle of a first arrangement.
FIG. 2 is a schematic diagram showing a concept of an example of a manufacturing method of a second arrangement.
FIG. 3 is a schematic diagram showing an example of the manufacturing method of the second arrangement.
FIG. 4 is a schematic diagram showing an example of a combination composition of a third arrangement.
FIG. 5 is a schematic diagram showing a comparative example (a) and an example (b) used in an experiment.
FIG. 6 is an image showing a result of the experiment.
FIG. 7 is an image showing a result of the experiment.
FIG. 8 is an image showing a result of the experiment.
FIG. 9 is a schematic diagram showing a comparative example (a) and an example (b) used in an experiment.
FIG. 10 is an image showing a result of the experiment.
FIG. 11 is an image showing a result of the experiment.
FIG. 12 is an image showing a result of the experiment.
FIG. 13 is an image showing a result of the experiment.
FIG. 14 is an image showing a result of the experiment.

DETAILED DESCRIPTION

According to one arrangement, radiotherapeutic lipid nanoparticles contain biodegradable lipid nanoparticles and a radioactive material as an active ingredient. The radioactive material is bound to lipid nanoparticles and is located outside of the lipid nanoparticles.

Hereinafter, arrangements will be explained with reference to the accompanying drawings. Note that, in each arrangement, same structural components will be referred to by the same reference numbers, and explanation thereof will be partially omitted. The figure is schematic, and the relationship between the thickness of each part and the plane dimensions, the ratio of the thickness of each part, etc., may differ from those in reality.

In the present application, "lipid nanoparticle" refers to a particle which is mainly composed of lipids. For example, forms commonly referred to as lipid nanoparticles (LNPs), liposomes, and microemulsions are also included in the term "lipid nanoparticle" herein.

(First arrangement)

Radiotherapeutic lipid nanoparticles according to an arrangement will be described referring to FIG. 1. A radiotherapy lipid nanoparticle 10 includes a biodegradable lipid nanoparticle 11 and a radioactive material 12 which is bound to the lipid nanoparticle 11 and located outside of the lipid nanoparticle. As to the lipid nanoparticle 11 and the radioactive material 12, for example, the radioactive material 12 may directly be bound to a portion of the lipid of the lipid nanoparticle 11, or may be bound through a mediating portion 15 of the lipid nanoparticle 11, such as, for example, a linking unit 15.

The linking unit 15 includes, for example, a linker 13 and nanoparticles 14 bound to the linker 13. The linker 13 may be a publically-known linker structure, which is bound to and/or extends from a functional group of any lipid of the lipid nanoparticle 11. For example, the linker 13 may be a portion of any lipid of the lipid nanoparticle 11, such as a portion of a

PEG-modified lipid, or a portion of a lipid provided with a functional group capable of binding a ligand, etc. FIG. 1 shows an example of the linker 13 which is a portion of PEG-modified lipid with a thiol group at the end, e.g., cholesterol. However, the linker 13 is not limited thereto.

As will be described in detail below, by including any one or more PEG-modified lipids or lipids provided with a functional group capable of binding ligands as lipid component of the lipid nanoparticles 11, a portion of those lipids can be used as the linker 13 when the lipid nanoparticles are formed as the lipid nanoparticles 11. Examples of functional groups to which the ligand can be bound include, but are not limited to, thiol groups, amino groups, maleimide groups, and carboxy groups.

Also, for example, the linker 13 may be included in the lipid material or lipid nanoparticle 11 formed in a molar ratio of 0.01 to 1% as the linker density. In other words, for example, if the amount of lipid used in a lipid nanoparticle is 1 mole, the amount of lipid of linker 13 contained therein would be 0.0001 to 0.01 mole. Using the linker 13 and nanoparticles 14, it is possible to stably bind the radioactive material 12 to the lipid nanoparticle 11 while positioning it outside thereof.

For example, the linker 13 is bound to a nanoparticle 14 which is capable of binding or has an affinity for the radioactive material to be used. As noted above, the example in FIG. 1 shows one example where the thiol group is present at the outer end of the linker 13. In this case, the nanoparticle 14 should be composed of a material which is capable of or has affinity for the thiol group and which is capable of or has affinity for the radioactive material 12. Such nanoparticles 14 may be, for example, Au, Ag, $SiO_2$, Si, glass, resin, etc., and may be nano-sized particles composed primarily of such materials. Furthermore, the surface of the nanoparticles may also be subjected to specific functionalization. Such functionalization may be, for example, active ester group modification, maleimide activation, thiol group modification, amino group modification, carboxy group modification, methyl group modification, avidin modification, and biotin modification. It can thereby facilitate linker binding and/or immobilization or binding of the radioactive material. The diameter ratio of the biodegradable lipid nanoparticle to the nanoparticle may be, for example, 1:1 to 100:1, 10:1 to 100:1, and 50:1 to 100:1. For example, one or more radioactive materials 12 are bound, attached or immobilized, for example, on the surface of such nanoparticles 14. In the example of FIG. 1, three radioactive materials 12 are immobilized on the surface of the nanoparticle 14, but the number is not limited thereto. The number of radioactive materials 12 bound to one lipid nanoparticle 11 may be one type or a combination of two or more types. In other words, the radioactive material 12 bound to one lipid nanoparticle 11 may contain one component or a plurality of components of different types from each other. Or, if multiple radiotherapy lipid nanoparticles are used for one system in which target cells to be targeted are present, different types of radioactive materials 12 may be selected and combined among the multiple radiotherapy lipid nanoparticles. The diameter of the particles may be, for example, 1 nm to 1 $\mu$m, 1 nm to 100 nm, or 1 nm to 10 nm.

The radioactive material 12 may be an artificial radioactive material, i.e., artificial radionuclide, or a natural radioactive material, i.e., natural radionuclide. Examples of radioactive materials include alpha-ray nuclides such as $^{211}$At, $^{213}$Bi, and $^{225}$Ac, beta-ray nuclides such as $^{89}$Sr, $^{186}$Re, $^{192}$Ir, $^{67}$Cu, $^{177}$Lu, $^{64}$Cu, $^{86}$Y, and $^{124}$I, or Auger electron emitter nuclides such as $^{67}$Ga, $^{111}$In, $^{114}$mIn, and $^{169}$Yb. They may be radioactive materials produced by accelerators, for example, alpha-ray nuclides, alpha emitters, etc. Examples of alpha emitters may be astatine ($^{211}$At), radium ($^{223}$Ra), and actinium ($^{225}$Ac). For example, an arrangement using astatine will be discussed below as a third arrangement.

A "target cell" may be a cell on which the radioactive material is to act. For example, a target cell may be a cancer cell, a proliferating cell, a cell affected by any other disease, or a damaged cell. Examples of cancer cells are metastatic cancer, blood cancers such as leukemia, ovarian cancer, thyroid cancer, pheochromocytoma, multiple myeloma, melanoma, glioma, leukemia, prostate cancer, breast cancer, ovarian cancer, etc. For example, the target cells may be selected according to the type of radioactive material used, the type or characteristics of the biodegradable lipid nanoparticles, the therapeutic target and/or the wishes of the practitioner. Application of the radiotherapy lipid nanoparticles to the target cells may be, for example, clinical, in laboratory, in vivo, in vitro, systemic, local, or any suitable route, such as intravascular, intraperitoneal, or organ administration.

The biodegradable lipid nanoparticles 11 may have target cell directivity. Here, "target cell directivity" means, for example, having an appropriate affinity for the target cell. Herein, "appropriate affinity" means having a higher affinity compared to similar transfection carriers of general design under normal and/or general contact conditions with the target cell and/or having a higher affinity for the target cell compared to affinity for cells other than the target cell. Target cell directivity, i.e., appropriate affinity, is achieved by adjusting the lipid composition of the biodegradable lipid nanoparticles.

The biodegradable lipid nanoparticles 11 include a lipid composition which exhibits the desired target cell directivity. The biodegradable lipid nanoparticle 11 is a sphere or abbreviated sphere formed of a lipid membrane, in other words, a hollow lipid nanoparticle. A biodegradable lipid nanoparticle is, in other words, a lipid nanoparticle. It may be a lipid membrane particle which encapsulates a core of aqueous solution, e.g., a lipid bilayer membrane particle. The lipid nanoparticle may be any publically-known lipid nanoparticle. For example, the lipid composition of the lipid nanoparticles may include, as its components, a first lipid of formula (I) (FFT-10) and/or a second lipid of formula (II) (FFT-20). These lipids are biodegradable lipids. By adjusting the lipid composition of the biodegradable lipid nanoparticles with these lipids, an appropriate affinity may be achieved.

[Formula 1]

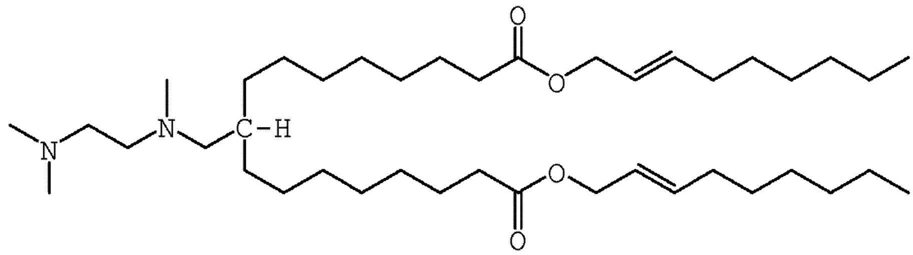

(I)

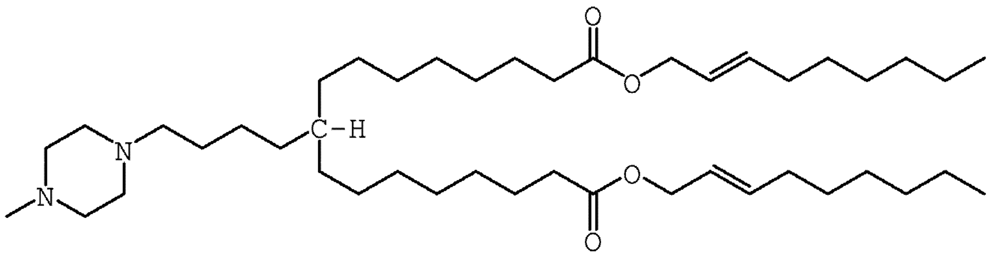

(II)

The biodegradable lipid nanoparticles, i.e., lipid nanoparticles, may contain further lipids in addition to the first and second lipids described above. Among the composition of the lipid molecular materials constituting the lipid nanoparticles, the fraction consisting of the first and second lipids is hereinafter referred to as "first fraction". The fraction consisting of lipid molecular materials other than the first or second lipid is hereinafter referred to as "second fraction". The lipids in the second fraction are hereinafter also referred to collectively as "third lipid".

The terms first and second fractions refer to the composition of the constituents of the lipid nanoparticles, not to the physical location of the lipids contained therein. For example, the components of the first and second fractions need not each be in one cohesive mass in the lipid nanoparticle, and the lipids in the first fraction can exist intermixed with those in the second fraction. The ratio of the first fraction to the total lipid material of the lipid nanoparticles can be 10% or more, 15% or more, 20% or more, 30% or more, 40% or more, 50% or less, 40% or less, 30% or less, 20% or less, for example, 10 to 50%, or 15 to 45%.

In other words, the total content of FFT-10 and/or FFT-20 as a percentage of lipid nanoparticles may be, for example, 10 to 50%, 10 to 45%, 10 to 40%, 10 to 35%, 10 to 30%, 10 to 25%, 10 to 20%, 15 to 50%, 15 to 45%, 15 to 40%, 10 to 35%, 15 to 30%, 15 to 25%, 15 to 20%, 20 to 50%, 20 to 45%, 20 to 40%, 20 to 35%, 20 to 30%, or 20 to 25%. The maximum content of FFT-10 and FFT-20 in the lipid nanoparticles may be, for example, the amount by which the lipid nanoparticles can form lipid nanoparticles. The percentage of the second lipid in the first fraction may be from 0 to 100%, for example, 15 to 75%, 20 to 60%, or 24 to 50%. Similarly, the percentage of the first lipid in the first fraction may be from 0% to 100%, for example, 15 to 75%, 20 to 60%, or 24 to 50%. Herein, percentages are expressed in moles/mol% unless otherwise specified.

The particle size and cell penetration of the lipid nanoparticles may change depending on the ratio of the first lipid to the second lipid in the first fraction. For example, when the second lipid increases, the particle size of the lipid nanoparticles may become larger. The average particle size of the lipid nanoparticles can be changed depending on the application. For example, it may be adjusted from about 20 to 300 nm. For example, it may be from about 50 to 100 nm.

The type of third lipid in the second fraction of lipid nanoparticles is not limited, but for example, the second fraction contains base lipids. The base lipid can be, for example, a lipid which is a major component of biological membranes. The base lipid may be a phospholipid or sphingolipid, such as diacylphosphatidylcholine, diacylphosphatidylethanolamine, ceramide, sphingomyelin, dihydrosphingomyelin, kephalin or cerebroside, or a combination thereof.

For example, the base lipid is the following: 1,2-dioleoyl-sn-glycero-3-phosphoethanolamine (DOPE), 1,2-stearoyl-sn-glycero-3-phosphoethanolamine (DSPE), 1,2-dipalmitoyl-sn-glycero-3-phosphatidylcholine (DPPC), 1-palmitoyl-2-oleoyl-sn-glycero-3-phosphatidylcholine (POPC), 1,2-di-O-octadecyl-3-trimethylammonium propane (DOTMA), 1,2-dioleoyl-3-dimethylammonium propane (DODAP), 1,2-dimyristoyl-3-dimethylammonium propane (14:0 DAP), 1,2-dipalmitoyl-3-dimethylammonium propane (16:0 DAP), 1,2-distearoyl-3-dimethylammonium propane (18:0 DAP), N-(4-carboxybenzyl)-N,N-dimethyl-2,3-bis(oleoyloxy)propane (DOBAQ), 1,2-dioleoyl-3-trimethylammonium propane (DOTAP), 1,2-dioleoyl-sn-glycero-3-phosphochlorin (DOPC), 1,2-dilinoleoyl-sn-glycero-3-phosphochlorin (DLPC), 1,2-dioleoyl-sn-glycero-3-phospho-L-serine (DOPS), or Cholesterol, or any combination of the above is preferred.

As the above base lipids, it is especially preferable to use cationic or neutral lipids, and the acid dissociation constant of the lipid nanoparticles can be adjusted by the content thereof. DOTAP is preferably used as the cationic lipid, and DOPE is preferably used as the neutral lipid.

The percentage of cationic lipids such as FFT10, FFT20, and DOTAP to the total lipid nanoparticles should be, for example, about 10 to 50% to adjust the appropriate affinity to the target cells. The percentage of cationic lipids to the total lipid nanoparticles may be, for example, 10 to 50%, 10 to 45%, 10 to 40%, 10 to 35%, 10 to 30%, 10 to 25%, 10 to 20%, 15 to 50%, 15 to 45%, 15 to 40%, 10 to 35%, 15 to 30%, 15 to 25 %, 15 to 20%, 20 to 50%, 20 to 45%, 20 to 40%, 20 to 35%, 20 to 30%, and 20 to 25%. In order to obtain an appropriate affinity for the target cell, lipid composition may be adjusted and designed by, for example, changing the component ratio of the cationic lipid to be included in the lipid nanoparticles or by having a gradient, depending on the type and condition of the target cell. For example, the component ratio of the cationic lipid may be adjusted to have an appropriate affinity for the target cells in a particular state.

The second fraction also preferably contains lipids which prevent aggregation of lipid nanoparticles. For example, lipids which prevent aggregation include PEG-modified lipids, such as polyethylene glycol (PEG) dimyristoylglycerol (DMG-PEG), omega-amino (oligoethylene glycol) alkanoic acid monomer derived from polyamide oligomers (US 6,320,017 B) or monosialogangliosides may be further included. The second fraction may further contain lipids such as relatively less toxic lipids to adjust for toxicity; lipids with functional groups which bind ligands to lipid nanoparticles; lipids to inhibit leakage of inclusions such as sterols, for example cholesterol. It is particularly desirable to include cholesterol.

The type and composition of the lipid used in the second fraction may be appropriately selected by considering the acid dissociation constant (pKa) of the target lipid nanoparticles, or the particle size of the lipid nanoparticles, or the type of active agent to be included, or stability in the cell.

One or more than one type of lipid from any of the above may be selected as desired for the linker 13. For example, the hydrophobic end of lipids such as DMG, DSPE, cholesterol, and DOPE may be modified by PEG or other modification to form the linker 13.

For example, further active agents may be included within the biodegradable lipid nanoparticles. The further active agent may be, for example, a further radioactive material, a substance having other pharmacological activity, or a nucleic acid construct encoding a gene. Further components may also be encapsulated as needed. Such components may be, for example, pH adjusters, osmotic pressure regulators, and gene activators. The pH adjusters are, for example, organic acids such as citric acid and their salts. The osmotic pressure regulators are, for example, sugars or amino acids. The gene activators are, for example, any substance which promotes or supports the activity of the active agent if the further active agent is a gene. Alternatively, for example, the biodegradable lipid nanoparticles may encapsulate a labeling substance which enables the radiotherapy lipid nanoparticles to be detected or visualized. For example, such a labeling substance may be another radioactive, fluorescent, dye, and chemiluminescent substance. For example, the above further substances and/or active agents may be one or a combination of two or more. Alternatively, such further substances and/or active agents may comprise a single component or a plurality of components of different types from each other.

Such radiotherapy lipid nanoparticles could provide a novel technique for delivering radioactive materials as active ingredients to target cells. It is also estimated to reduce side effects derived from the delivery system.

(Second arrangement)

One example of a manufacturing method of radiotherapy lipid nanoparticles 10 of a second arrangement will be described referring to FIGS. 2 and 3. First, a lipid material having the desired lipid composition is used to form lipid nanoparticles 11, for example, by the publically-known method as described above (part (a) of FIG. 2). At least a portion of the lipid material used herein should be modified such that the lipid nanoparticles 11 contain the desired linker 13, for example. The nanoparticles 14, which can be immobilized at the end of the linker 13, are added thereto and stirred for incubation (part (a) of FIG. 2). The radioactive material 12 is further added, stirred, and incubated (part (b) of FIG. 2). This yields lipid nanoparticles 10 for radiotherapy (part (c) of FIG. 2). Incubation may be performed here, for example, by leaving them under a constant temperature. These processes can be rephrased as follows. That is, the method of manufacturing the radiotherapy lipid nanoparticle 10 includes preparing biodegradable lipid nanoparticles 11 (part (a) of FIG. 2, FIG. 3 (S31)), mixing biodegradable lipid nanoparticles 11 and radionuclides 12 (part (b) of FIG. 2, FIG. 3 (S32)), incubating the resulting mixture (part (b) of FIG. 2, FIG. 3 (S33)), and obtaining the radiotherapy lipid nanoparticle 10 (part (c) of FIG. 2, FIG. 3 (S34)).

Preparation of biodegradable lipid nanoparticles 11 may be performed, for example, by the formation of lipid nanoparticles using the desired materials by the Bangham method, organic solvent extraction, surfactant removal, or freeze-thaw method. For example, lipid nanoparticles may be formed by preparing a lipid mixture obtained by including the biodegradable lipid nanoparticle material in a desired ratio in an organic solvent such as alcohol and an aqueous buffer,

adding the aqueous buffer to the lipid mixture, and stirring and suspending the resulting mixture. The lipid nanoparticles obtained as above are one example of biodegradable lipid nanoparticles 11. For example, encapsulation of further active agents or further components in the lipid nanoparticles 11 may be achieved by including the components to be encapsulated in the aqueous buffer solution described above.

For example, the conditions of incubation may be selected according to the nature of the radioactive substance used and under pharmaceutically acceptable conditions. For example, incubation may be performed at temperature conditions of about 4 to 37°C for about 10 minutes to about 1 hour. Alternatively, for example, incubation may be performed by leaving at room temperature.

Obtaining the radiotherapy lipid nanoparticles 10 includes a process of forming desired radiotherapy lipid nanoparticles 10. Further processes may be included if desired. For example, it may further include isolating formed radiotherapy lipid nanoparticles 10, and it may further include washing the resulting radiotherapy lipid nanoparticles 10.

According to the above manufacturing method of radiotherapy lipid nanoparticles 10, it is possible to provide a novel technique for delivering a radioactive material as an active ingredient to target cells. It is possible to more easily obtain target cell-directed lipid nanoparticles as a means of delivering a radioactive material to target cells.

(Third arrangement)

Radiotherapy lipid nanoparticles according to a third arrangement are similar to the radiotherapy lipid nanoparticles according to the first arrangement, except that they contain astatine ($^{211}At$) as a radioactive material. The specific configuration will be described referring to FIG. 1. The radiotherapy lipid nanoparticle 10 includes a biodegradable lipid nanoparticle 11 and astatine as a radioactive material 12 which is bound to the lipid nanoparticle 11 and located outside of the lipid nanoparticle. The binding of the lipid nanoparticles 11 to the astatine 12 is achieved by a linking unit 15. The linking unit 15 has, for example, a linker 13 and a nanoparticle 14 bound to the linker 13. For example, the linker 13 may be a lipid which is PEG-modified and has a thiol group at the end, such as DSPE and/or cholesterol. The nanoparticles 14 may be, for example, Au nanoparticles. The mode of binding of astatine to Au is as shown in Number 1 below and in Tables 1 and 2.

[Number 1]

$$At^- + Au_n + H_2O \rightarrow Au_nAt + \frac{1}{2}H_2 + OH^-$$

[Table 1]

| | | | | | |
|---|---|---|---|---|---|
| $Au_{12}At$ Series | 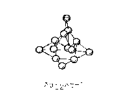 | 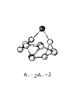 | 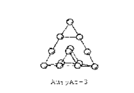 | 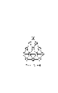 | |
| $Au_{13}At$ Series | 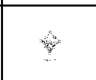 | 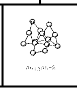 | 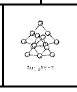 | 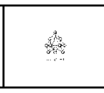 | 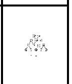 |
| "On AunAt clusters as potential astatine carriers", *RCS Advances,* **2017** | | | | | |

[Table 2]

Table 2

| System | X F | Cl | Br | I | At |
|---|---|---|---|---|---|
| $X_2+2e \rightarrow 2X^-$ | -66.09 | -33.90 | -26.75 | -16.60 | -6.00 |
| AuX | -66.18 | -75.08 | -75.61 | -78.38 | -80.20 |
| $Au_{12}X$-1 | -13.51 | -22.91 | -22.28 | -28.15 | -26.46 |
| $Au_{12}X$-2 | -15.11 | -25.71 | -26.88 | -34.35 | -33.16 |
| $Au_{12}X$-3 | -41.21 | -49.41 | -54.18 | -58.45 | -55.46 |
| $Au_{12}X$-4 | -13.21 | -21.21 | -20.18 | -24.85 | -22.26 |

(continued)

| | X | | | | |
|---|---|---|---|---|---|
| System | F | Cl | Br | I | At |
| $Au_{13}X$-1 | -54.21 | -61.51 | -60.88 | -65.45 | -67.56 |
| $Au_{13}X$-2 | -51.11 | -60.21 | -59.48 | -64.45 | -67.06 |
| $Au_{13}X$-3 | -69.01 | -78.11 | -77.28 | -82.25 | -83.56 |
| $Au_{13}X$-4 | -44.21 | -53.61 | -52.78 | -57.95 | -60.56 |
| $Au_{13}X$-5 | 2.19 | -4.91 | -2.28 | -5.15 | -6.46 |
| "On AunAt clusters as potential astatine carriers", RCS Advances, 2017 | | | | | |

Astatine is an alpha emitter and a small amount can provide a strong therapeutic effect. For example, a target cell of the radiotherapy lipid nanoparticle 10 containing astatine ($^{211}$At) as an active ingredient may be, for example, metastatic cancer. By utilizing biodegradable lipid nanoparticles 11 which are target cell directed, the side effects of astatine on normal cells can be suppressed. Because astatine has a short half-life of approximately 7 hours, when using lipid nanoparticles for radiotherapy containing astatine as an active ingredient for treatment, the binding of the lipid nanoparticles 11 to astatine 12 should occur immediately prior to administration to the patient. In that case, astatine and the biodegradable lipid nanoparticles 11 may be provided as a combination composition for radiation therapy, as described below.

The production of astatine may be performed by any of publically-known methods. For example, the production method may involve three processes: transmutation, separation and recovery, and synthesis. Specifically, for example, the process may be carried out as follows. First, in transmutation, 4$He^{2+}$ ions accelerated to about 28 MeV by an accelerator are irradiated to a bismuth target to produce $^{211}$At by the $^{209}$Bi($\alpha$,2 n)$^{211}$At reaction.

The separation and recovery process are as follows. Since the raw material $^{209}$Bi and $^{211}$At produced by nuclear reaction are mixed in the target after irradiation, the two are separated and only At is recovered. As an example of the separation method, the Bi target is placed in a quartz tube, heated to 850°C in an electric furnace, and the $^{211}$At, which has become a gas, is passed through a fluoroplastic tube cooled to -100°C with an oxygen stream to solidify the $^{211}$At, which is collected on the inner wall of the tube. Alternatively, a wet method of dissolving the $^{211}$At with an acid or the like may be used.

The form of recovery may vary depending on the separation method. For example, $^{211}$At which is finally collected in a quartz tube or filter may be washed with a washing solution and recovered in a solution form. The recovered solution may be used to produce radiotherapy lipid nanoparticles by chemically combining $^{211}$At with biodegradable lipid nanoparticles designed for the cancer to be treated by a synthesizer.

For the production method of astatine, refer to related literatures, e.g., "Development of a Mass Production Method of Artificial Element Astatine - Accelerating the Development of Cancer Therapeutics Using Alpha Radiation" (https: /www.riken.jp/press/2023/20230831_3/index.html) and "Separation of At-211 by Dry and Wet Methods," Shigeki Watanabe et al., 15th Annual Meeting on Radiopharmaceuticals and Imaging Agents (2015).

The route of application of astatine-containing radiotherapy lipid nanoparticles to target cells may be, but not limited to, for example, intravenous administration. Thereby, they can be delivered to lesions such as cancer metastases.

The radiotherapy lipid nanoparticles containing astatine as an active ingredient would provide a novel technology for delivering radioactive materials as active ingredients to target cells. It is also estimated to reduce side effects derived from the delivery system. Alpha emitters such as astatine can produce strong therapeutic effects at small doses. In addition, the use of lipid nanoparticles with target cell directivity allows for efficient delivery to target cells while preventing the effects on normal cells during treatment, which will cause side effects. It is also relatively easy to adjust the directivity to the desired tumor cells by changing the lipid composition of the lipid nanoparticles. In the past, it was necessary to select antibodies or to develop new antibodies for each disease, limiting the scope of application; however, with the radiotherapy lipid nanoparticles containing astatine of the arrangement, easier design and broader application as compared to the conventional cases can be expected.

(Fourth arrangement)

The radiotherapy lipid nanoparticle according to the first and third arrangements described above may be provided ready for immediate use on the desired target cells, or may be provided as a radiotherapy lipid nanoparticle manufacturing kit in the form of a material which can be adjusted at the time of use by the user of the radiotherapy lipid nanoparticle described above. In that case, as shown in FIG. 4, the kit may have, for example, biodegradable lipid nanoparticles 11 or biodegradable lipid nanoparticle material (not shown) configured to be directed to a desired target cell, and a radioactive material 12 (part (a) of FIG. 4). Alternatively, a portion of the composition of the biodegradable lipid

nanoparticle 11, e.g., nanoparticles 14, may be provided in a form independent of the lipid nanoparticles (part (b) of FIG. 4). For example, the manufacturing kit of the radiotherapy lipid nanoparticles independently includes biodegradable lipid nanoparticles for the radiotherapy lipid nanoparticle and a radioactive material as an active ingredient. For example, "independently includes" means that they are stored in different containers as a first component and a second component, respectively. The first and second components stored in different containers may be delivered to the user, for example, co-housed in a single box or other container, or the first and second components may be delivered separately to the user from different sources.

As a manufacturing kit of radiotherapy lipid nanoparticles provided as above, it is easier to handle each composition or component in an appropriate environment.

(Fifth arrangement)

The radiotherapy lipid nanoparticles according to the first and third arrangements described above, and the radiotherapy lipid nanoparticle manufacturing kit according to the fourth arrangement, may be provided as a composition ready for immediate use on the desired target cells (e.g., a pharmaceutical composition), or as a combination composition (e.g., a combination pharmaceutical composition) which is adjusted immediately before use by the user of the radiotherapy lipid nanoparticles described above. For example, if the lipid nanoparticles or lipid nanoparticle manufacturing kit is provided as a combination composition, the combination composition may include, for example, a first composition containing biodegradable lipid nanoparticles 11 or biodegradable lipid nanoparticle material (not shown) configured to be directed to a desired target cell and a second composition containing a radioactive material 12 (part (a) of FIG. 4). Alternatively, the combination composition may have a first composition containing base lipid nanoparticles 11, a second composition containing the radioactive material 12, and furthermore, a third composition containing a portion of the linking portion 15 (e.g., nanoparticles 14) which is part of the composition of the biodegradable lipid nanoparticles 11 (part (b) of FIG. 4). The compositions and combination compositions may include particle-bound lipid nanoparticles, e.g., cell surface-staying microparticle-bound lipid nanoparticles, and may further have components and/or compositions publically-known as desired. For example, the ingredients and/or compositions may be selected to be pharmacologically and/or medically stable, or by which necessary and sufficient conditions are physically and/or chemically and/or pharmacologically and/or medically met.

The composition or combination composition may be a pharmaceutical composition. For example, the composition or combination composition includes a radiotherapy lipid nanoparticle or radiotherapy lipid nanoparticle material in a pharmaceutically acceptable state and/or as an ingredient for delivering a radioactive material as an active ingredient to a target cell. The composition may be used in clinical or non-clinical fields. Such compositions or combination compositions may include appropriate additives, e.g., stabilizers, pH adjusters, buffers, viscosity adjusters, and excipients, depending on the desired method of use, route of administration, target cells to be used and subject to be administered, respectively. For example, when such compositions or combination compositions are provided as pharmaceutical compositions to be administered to a subject, the ingredients included are selected and designed within pharmaceutically acceptable limits. For example, a combination composition containing radiotherapy lipid nanoparticles may be a combination composition with a first composition containing biodegradable lipid nanoparticles for radio therapy and a second composition containing a radioactive material as an active ingredient to be bound to the lipid nanoparticles. The biodegradable lipid nanoparticles may further include a linker extending to the end of a portion of the component lipid and nanoparticles immobilized on the linker to immobilize said radioactive material. The first and second compositions may be provided in first and second containers, respectively, and the first and second containers may be further contained in a third container. Alternatively, the first and second compositions may be accommodated in the first and second containers, respectively, and provided independently of each other. In that case, for example, they may be provided respectively to the user from different manufacturers or providers.

The compositions or combination compositions as above could provide novel techniques for delivering radioactive materials as active ingredients to target cells. It is also estimated to reduce side effects derived from the delivery system.

[Example 1]

Hereinafter, an experimental example in which a drug delivery system which mimics lipid nanoparticles for radiotherapy is constructed according to the arrangement and its target cell directivity is investigated will be explained.

Experiment 1: Preparation of target cell-directed lipid nanoparticles

Biodegradable lipid nanoparticles were prepared and fluorescent lipids were used as a substitute for the radioactive material for the outside of the lipids of such lipid nanoparticles. Specifically, as shown in part (b) of FIG. 5,

radiotherapy lipid nanoparticles 50 include biodegradable lipid nanoparticles 11, a linker 13 composed by modification to a portion of the lipid of the lipid nanoparticles 11 and a bead 61 with streptavidin attached to the surface thereof as a microparticle bound to the linker 13 (part (b) of FIG. 5). Target cell tropism was tested on this model. The fluorescent material used was Rhodamine-PE (Avanti). Resin beads 61 ($\varphi$ = 3 $\mu$m, Bang) were prepared for comparison (part (a) of FIG. 5).

The materials for the biodegradable lipid nanoparticles were FFT-20, DOPE, DOTAP, cholesterol, DSPE-PEG2000-Biotin, and Rhodamine-PE in molar ratios of 31.7:4.5:9.0:51.4:3.4:0.1 respectively. These materials were dissolved in ethanol to obtain a lipid solution. The lipid solution was mixed with the above 10 mM HEPES (pH 7.3) solution using a microflowchip and syringe pump. The mixed solution was further diluted 10-fold with 10 mM HEPES (pH 7.3) and concentrated with an ultrafiltration filter (Amicon Ultra 0.5 Ultracel-50, Merck) to obtain biodegradable lipid nanoparticles 11 with the linker 13. Streptavidin beads were then added in buffer solution to obtain the radiotherapy lipid nanoparticle model 50 of Example 1.

Experiment 2: Visualization of gold nanoparticles binding to lipid nanoparticles

A streptavidin beads suspension (Comparative Example 1) was prepared in a micro tube, to which buffer was added as a control and centrifuged. The image obtained by photographing the precipitates is shown in part (a) of FIG. 6). The radiotherapy lipid nanoparticle model 50 prepared in Experiment 1 (Example 1) was added to the microtubes and centrifuged. The image obtained by photographing the state of the precipitation obtained is shown in part (b) of FIG. 6. The color of the streptavidin beads exhibited the light pink color of Rhodamine-PE, indicating that the lipid nanoparticles were bound to the avidin beads.

Experiment 3: Observation under a microscope

Streptavidin beads of Comparative Example 1 and radiotherapy lipid nanoparticles model 50 of Example 1 were added to culture dishes and observed under a microscope in bright field and fluorescent field, respectively, as shown in FIG. 7. In the bright field, both Comparative Example 1 and Example 1 were observed to be dispersed. However, in the fluorescent field of view, only the fluorescent material provided by the radiotherapy lipid nanoparticle model 50 of Example 1 was observed.

Experiment 4: Study of target cell tropism with respect to normal and cancer cell models

The radiotherapy lipid nanoparticle model 50 of Example 1 was suspended in buffer solution (composition: pH 7.4 HEPES solution with 200 mM glucose dissolved) and added to two giant unilamellar vesicles (GUVs) as models of normal cells and cancer cells, respectively, and incubated at 37°C for 10 minutes. Then, they were observed under a fluorescence microscope after the incubation. The GUV of normal cells is formed with a lipid composition containing only DOPC, while the GUV as a model of cancer cells is formed of a lipid composition containing DOPC:DOPS:DOPE in the ratio of 8:1:1. Results of mixing the GUVs and the lipid nanoparticles are shown in FIG. 8. In the GUVs of normal cells, it was observed that the radiotherapy lipid nanoparticle model 50 and the cells were independent of each other (part (a) of FIG. 8). In contrast, the radiotherapy lipid nanoparticle model 50 were observed to bind to the surface of cancer cell GUVs (part (b) of FIG. 8).

[Example 2]

Next, an experimental example in which biodegradable lipid nanoparticles containing gold nanoparticles and a terminally extended linker for immobilizing radioactive materials were manufactured, and the effect of adding gold nanoparticles on cell transduction properties was investigated, will be explained.

Experiment 5: Preparation of gold nanoparticle-bound lipid nanoparticles

Biodegradable lipid nanoparticles with gold nanoparticles bound thereto were prepared. Specifically, as shown in part (b) of FIG. 9, biodegradable lipid nanoparticles 70 have biodegradable lipid nanoparticles 11, nucleic acid 82 encapsulated in the lipid nanoparticles 11, linker 13 composed by modification to a portion of the lipid of lipid nanoparticles 11, and gold nanoparticles 81 as nanoparticles bound to the linker 13 (part (b) of FIG. 9). As a comparison, biodegradable lipid nanoparticles 71 were prepared without gold nanoparticles and linker (part (a) of FIG. 9).

Experiments were performed on two different compositions (hereafter referred to as B and C). The materials for biodegradable lipid nanoparticles 70 (B) were FFT-20, DOPE, DOTAP, cholesterol, DMG-PEG2000, and DMG-PEG2000-Thiol in molar ratios of 31.7:4.5:9.0:51.4:3.1:0.3, respectively. The materials for biodegradable lipid nanoparticles 70(c)

were FFT-20, DOPE, DOTAP, cholesterol, DMG-PEG2000, and DMG-PEG2000-Thiol in molar ratios of 31.7:4.5:9.0:51.4:2.1:1.3 respectively. That is, the lipid nanoparticle B contains DMG-PEG2000 and DMG-PEG2000-Thiol in a molar ratio of 9:1, and the lipid nanoparticle C contains DMG-PEG2000 and DMG -PEG2000-Thiol in a molar ratio of 7:3. These materials were dissolved in ethanol to obtain lipid solutions. 1 mg/ml of GFP-mRNA (TriLink BioTechnologies, about 1000 bp) and 10 mM HEPES (pH 7.3) solution were mixed at a volume ratio of 1:9 to obtain nucleic acid solutions. The nucleic acid solution was the same for all lipid nanoparticles. The lipid solution and the above nucleic acid solution were mixed at a ratio of 1:1 using a microflowchip and syringe pump, and then the mixed solution was diluted 4-fold with 10 mM HEPES (pH 7.3), and then, gold nanoparticles (Nanoprobes, Inc., diameter 1.9 nm) were added in buffer solution and allowed to react at room temperature for 30 minutes. The biodegradable lipid nanoparticles 70 of Example 2 were concentrated on an ultrafiltration filter (Amicon Ultra 0.5 Ultracel-50, Merck).

The materials for biodegradable lipid nanoparticles 71 (A) in Comparative Example 2 were FFT-20, DOPE, DOTAP, cholesterol, DMG-PEG2000, and DMG-PEG2000-Thiol in a molar ratio of 31.7:4.5:9.0:51.4:3.1:0.3, respectively. The lipid solution and the nucleic acid solution were mixed using a microflowchip and syringe pump. The mixed solution was diluted 10-fold with 10 mM HEPES (pH 7.3) and concentrated with an ultrafiltration filter (Amicon Ultra 0.5 Ultracel-50, Merck) to obtain biodegradable lipid nanoparticles 71 containing nucleic acids 82.

Experiment 6: Observation under electron microscope

FIG. 10 shows the results of observation of biodegradable lipid nanoparticles 71 (A) of Comparative Example 2 and 70 (C) of Example 2, each encapsulating nucleic acids 82, after negative staining using uranyl acetate solution. The lipid nanoparticles were observed to be dispersed in both Comparative Example 2 and Example 2. In addition, in Example 2, black dots indicating gold nanoparticles were observed on the outside of the biodegradable lipid nanoparticles 70, suggesting that the gold nanoparticles 81 could be bound to the surface of lipid nanoparticles 11 via linker 13.

Experiment 7: Introduction of lipid nanoparticles into breast cancer cell lines

The introduction was performed into cell lines seeded in 96-well plates at a cell count of $2 \times 10^5$. The lipid nanoparticles 71 (A) of Comparative Example 2 and biodegradable lipid nanoparticles 70 (B) and (C) of Example 2, each encapsulating nucleic acids 82, were introduced into the breast cancer cell line MCF-7, and the results of fluorescence observation under a microscope after 24 hours of culture are shown in part (a) of FIG. 11. Samples of the cell line alone, to which no lipid nanoparticles were added, are shown as "no-liposomes". The lipid nanoparticles that includes total 200 ng of nucleic acid was added to each sample. Both Comparative Example 2 and Example 2 showed similar levels of GFP fluorescence from the breast cancer cell lines, and the cells showed almost the same morphology. The values of GFP fluorescence intensity of MCF-7 measured by a plate reader are shown in part (b) of FIG. 11. The fluorescence intensity values of A, B, and C for each lipid nanoparticle were calculated by subtracting the fluorescence intensity value of no-liposome from the fluorescence intensity value of cells transfected with each lipid nanoparticle; no significant differences in fluorescence were observed among cells transfected with the lipid nanoparticles A, B, and C. These results suggest that the lipid nanoparticles bound with gold nanoparticles have the same tropism as the biodegradable lipid nanoparticles 71 without gold nanoparticles and linkers, but no difference in toxicity.

Experiment 8: Introduction of lipid nanoparticles into liver cancer-derived cell lines

The results of introducing biodegradable lipid nanoparticles 71 (A) of Comparative Example 2, biodegradable lipid nanoparticles 70 (B) and 70 (C) of Example 2, each encapsulating 82 nucleic acids, into the human liver cancer derived cell line Huh-7 are shown in FIG. 12. All conditions, except for the cell type, were performed in the same manner as in Experiment 6. As in Experiment 6, no significant differences were observed in the fluorescence photograph and plate reader results. Therefore, it was suggested that for the same composition, the tropism and toxicity trends were similar even if the cell type was changed.

Experiment 9: Verification of cell-tropism of biodegradable lipid nanoparticles with different lipid compositions

Whether cell tropism is maintained for biodegradable lipid nanoparticles with two lipid compositions (hereinafter referred to as D and E) different from lipid compositions A and B in Experiment 8, regardless of the type of ionized lipid in the components thereof, were verified. First, four types of lipid nanoparticles were prepared: biodegradable lipid nanoparticles 70 (D) and 71 (E) encapsulating nucleic acids 82, respectively, in addition to the biodegradable lipid nanoparticles 70 (B) and 71 (A) prepared in Experiment 8. The material of biodegradable lipid nanoparticle 71 (D) was a lipid solution mixed with FFT-10, DOTAP, cholesterol, DMG-PEG2000, and DMG-PEG2000-Thiol in the mole fraction of 31.7:26.6:9.0:38.0:2.2:0.3 respectively. The gold nanoparticles were reacted under the same conditions as in Experiment

5 to obtain biodegradable lipid nanoparticles 71 (D). The material of biodegradable lipid nanoparticles 71 (E) is a lipid solution mixed with FFT-10, DOTAP, cholesterol, and DMG-PEG2000 in a mole fraction of 31.7:26.6:9.0:38.0:2.5, respectively. Each of the four types of lipid nanoparticles was introduced into breast cancer cell lines MDA-MB231 or human liver cancer-derived cell lines Huh7 for verification of cell tropism. MDA-MB231 cells were seeded in 96-well plates at a cell number of $2 \times 10^5$, and a total of 200 ng of nucleic acid was introduced into each lipid nanoparticle. Huh7 cells were seeded in 96-well plates at $1 \times 10^5$ cells, and each lipid nanoparticle was introduced into the cells at a total of 100 ng of endogenous nucleic acid.

Fluorescence micrographs of each cell type with each lipid nanoparticle introduced therein 2 hours after introduction are shown in FIG. 13. Observations were performed with a 20x objective lens. The fluorescence intensity values of GFP measured by a plate reader are also shown in FIG. 14. The value of each fluorescence intensity was calculated by subtracting the value of the fluorescence intensity of cells without lipid nanoparticles (i.e., background fluorescence intensity) from the fluorescence intensity of cells with each lipid nanoparticle introduced (n = 3, error bars indicate standard error) by a plate reader. FIGS. 13 and 14 show that both compositions (D) and (E) were directed toward Huh7, and both compositions (B) and (A) were directed toward MDA-MB231. It was shown that the lipid nanoparticles can be given tropism to different cell types by changing the lipid composition. In addition, it is understood that the tropism of the lipid nanoparticles to target cells is independent of the presence or absence of nanoparticles, such as gold nanoparticles, in other words, the tropism of the lipid nanoparticles to target cells is maintained even when nanoparticles are bound. In other words, it is possible to achieve both surface modification of nanoparticles and cell delivery, regardless of the type of ionized lipid such as FFT-10 or FFT-20.

These results suggest that lipid nanoparticles with target cell tropism can be used to provide novel radiotherapy lipid nanoparticles for the delivery of radioactive material as an active agent to target cells.

While certain arrangements have been described, these arrangements have been presented by way of example only, and are not intended to limit the scope of the claims. Indeed, the radiotherapy lipid nanoparticle, combination composition, kit and method described herein may be embodied in a variety of other forms; furthermore, various omissions, substitutions and changes in the form of the radiotherapy lipid nanoparticle, combination composition, kit and method described herein may be made.

The arrangements as described above include clauses below.

Clause 1
A radiotherapy lipid nanoparticle (10) characterized by including a biodegradable lipid nanoparticle (11), and a radioactive material (12) as an active ingredient bound to the biodegradable lipid nanoparticle and located outside of the biodegradable lipid nanoparticle.

Clause 2
The radiotherapy lipid nanoparticle of clause 1, characterized in that the biodegradable lipid nanoparticle further includes a linker (13) extending to an end of a portion of component lipid thereof and a nanoparticle (14) immobilized on the linker to immobilize the radioactive material.

Clause 3
The radiotherapy lipid nanoparticle of clause 1 or clause 2, characterized in that the biodegradable lipid nanoparticle has a lipid composition which exhibits target cell tropism.

Clause 4
The radiotherapy lipid nanoparticle of any one of clause 1 to clause 3, characterized in that the lipid composition of the biodegradable lipid nanoparticle contains cationic lipids at 10 to 50%.

Clause 5
The radiotherapy lipid nanoparticle of any one of clause 1 to clause 4, characterized in that the lipid composition of the biodegradable lipid nanoparticle contains FFT10 and FFT20 at 10 to 50% of the total lipid content.

Clause 6
The radiotherapy lipid nanoparticle of any one of clause 1 to clause 5, characterized in that the biodegradable lipid nanoparticle encapsulate a labeling substance which makes the radiotherapy lipid nanoparticle detectable.

Clause 7
The radiotherapy lipid nanoparticle of any one of clause 2 to clause 6, characterized in that the linker is included in the biodegradable lipid nanoparticle at a specific linker density.

Clause 8
The radiotherapy lipid nanoparticle of any one of clause 2 to clause 7, characterized in that major components of the nanoparticle are Au, Ag, $SiO_2$, Si, glass, and resin.

Clause 9
The radiotherapy lipid nanoparticle of any one of clause 2 to clause 8, characterized in that a surface of the nanoparticle is functionalized.

Clause 10

The radiotherapy lipid nanoparticle of any one of clause 2 to clause 9, characterized in that the diameter ratio of the biodegradable lipid nanoparticle to the nanoparticle is 1:1 to 100:1.
Clause 11
The radiotherapy lipid nanoparticle of any one of clause 1 to clause 10, characterized in that the radioactive material is an alpha emitter.
Clause 12
The radiotherapy lipid nanoparticle of any one of clause 1 to clause 11, characterized in that the radioactive material is astatine.
Clause 13
A method of manufacturing the radiotherapy lipid nanoparticle of any one of clause 1 to clause 12, the method characterized by including

preparing the biodegradable lipid nanoparticle, mixing the biodegradable lipid nanoparticle and the radioactive material,
incubating a mixture obtained, and
obtaining a radiotherapy lipid nanoparticle.

Clause 14
The method of clause 13, characterized in that the biodegradable lipid nanoparticle further includes a linker extending to an end of a portion of component lipid thereof and a nanoparticle immobilized on the linker to immobilize the radioactive material.
Clause 15
The method of clause 13 or clause 14, characterized in that the radioactive material is an alpha emitter.
Clause 16
The method of any one of clause 13 to clause 15, characterized in that the radioactive material is astatine.
Clause 17
A combination composition including a first composition containing a biodegradable lipid nanoparticle for a radiotherapy lipid nanoparticle and a second composition containing a radioactive material as an active ingredient for binding to the lipid nanoparticle.
Clause 18
The combination composition of clause 17, characterized in that the biodegradable lipid nanoparticle further includes a linker extending to an end of a portion of component lipid thereof and a nanoparticle immobilized on the linker to immobilize the radioactive material.
Clause 19
The combination composition of clause 17 or clause 18, characterized in that the radioactive material is an alpha emitter.
Clause 20
The combination composition of any one of clause 17 to clause 19, characterized in that the radioactive material is astatine.
Clause 21
A radiotherapy lipid nanoparticle manufacturing kit including a biodegradable lipid nanoparticle for a radiotherapy lipid nanoparticle and a radioactive material as an active ingredient, which are included independently.
Clause 22
The kit of clause 21, characterized in that the biodegradable lipid nanoparticle further includes a linker extending to an end of a portion of component lipid thereof and a nanoparticle immobilized on the linker to immobilize the radioactive material.
Clause 23
The kit of clause 21 or clause 22, characterized in that the radioactive material is an alpha emitter.
Clause 24
The kit of any one of clause 21 to clause 23, characterized in that the radioactive material is astatine.

## Claims

**1.** A radiotherapy lipid nanoparticle (10), **characterized by** comprising:

a biodegradable lipid nanoparticle (11); and
a radioactive material (12) as an active ingredient bound to the biodegradable lipid nanoparticle and located

outside of the biodegradable lipid nanoparticle.

2. The radiotherapy lipid nanoparticle of claim 1, **characterized in that** the biodegradable lipid nanoparticle further includes a linker (13) extending to an end of a portion of component lipid thereof, and a nanoparticle (14) immobilized on the linker to immobilize the radioactive material.

3. The radiotherapy lipid nanoparticle of claim 1, **characterized in that** the biodegradable lipid nanoparticle has a lipid composition which exhibits target cell tropism.

4. The radiotherapy lipid nanoparticle of claim 1, **characterized in that** a lipid composition of the biodegradable lipid nanoparticle contains cationic lipids at 10 to 50%.

5. The radiotherapy lipid nanoparticle of claim 1, **characterized in that** the lipid composition of the biodegradable lipid nanoparticle contains FFT10 and FFT20 at 10 to 50% of the total lipid content.

6. The radiotherapy lipid nanoparticle of claim 1, **characterized in that** the biodegradable lipid nanoparticle encapsulate a labeling substance by which the radiotherapy lipid nanoparticle becomes detectable.

7. The radiotherapy lipid nanoparticle of claim 2, **characterized in that** the linker is included in the biodegradable lipid nanoparticle in a molar ratio of 0.01 to 1% of the component molecules.

8. The radiotherapy lipid nanoparticle of claim 2, **characterized in that** major components of the nanoparticle are Au, Ag, $SiO_2$, Si, glass, and resin.

9. The radiotherapy lipid nanoparticle of claim 2, **characterized in that** a surface of the nanoparticle is functionalized.

10. The radiotherapy lipid nanoparticle of claim 2, **characterized in that** a diameter ratio of the biodegradable lipid nanoparticle to the nanoparticle is 1:1 to 100:1.

11. The radiotherapy lipid nanoparticle of claim 1, **characterized in that** the radioactive material is an alpha emitter.

12. A method of manufacturing the radiotherapy lipid nanoparticle of claims 1, the method **characterized by** comprising:

    preparing the biodegradable lipid nanoparticle;
    mixing the biodegradable lipid nanoparticle and the radioactive material;
    incubating a mixture obtained; and
    obtaining a radiotherapy lipid nanoparticle.

13. The method of claim 12, **characterized in that** the biodegradable lipid nanoparticle further includes a linker extending to an end of a portion of component lipid thereof and a nanoparticle immobilized on the linker to immobilize the radioactive material.

14. A kit for manufacturing the radiotherapy lipid nanoparticle of claim 1,
    the kit **characterized by** including a biodegradable lipid nanoparticle for a radiotherapy lipid nanoparticle and a radioactive material as an active ingredient, which are included independently.

15. The kit of claim 14, **characterized in that** the biodegradable lipid nanoparticle further includes a linker extending to an end of a portion of component lipid thereof and a nanoparticle immobilized on the linker to immobilize the radioactive material.

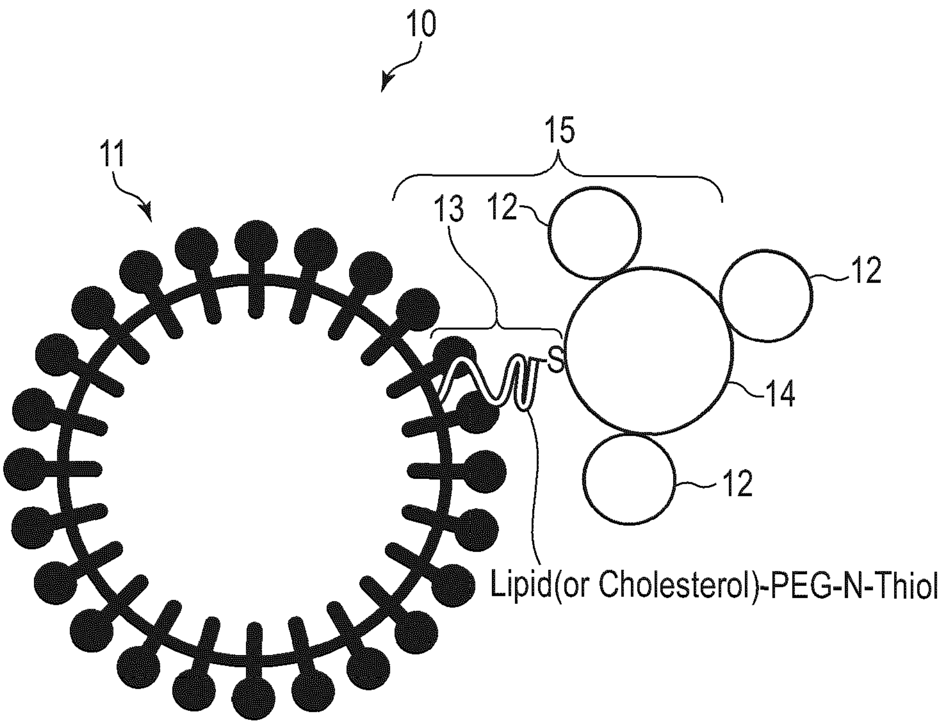


FIG. 1

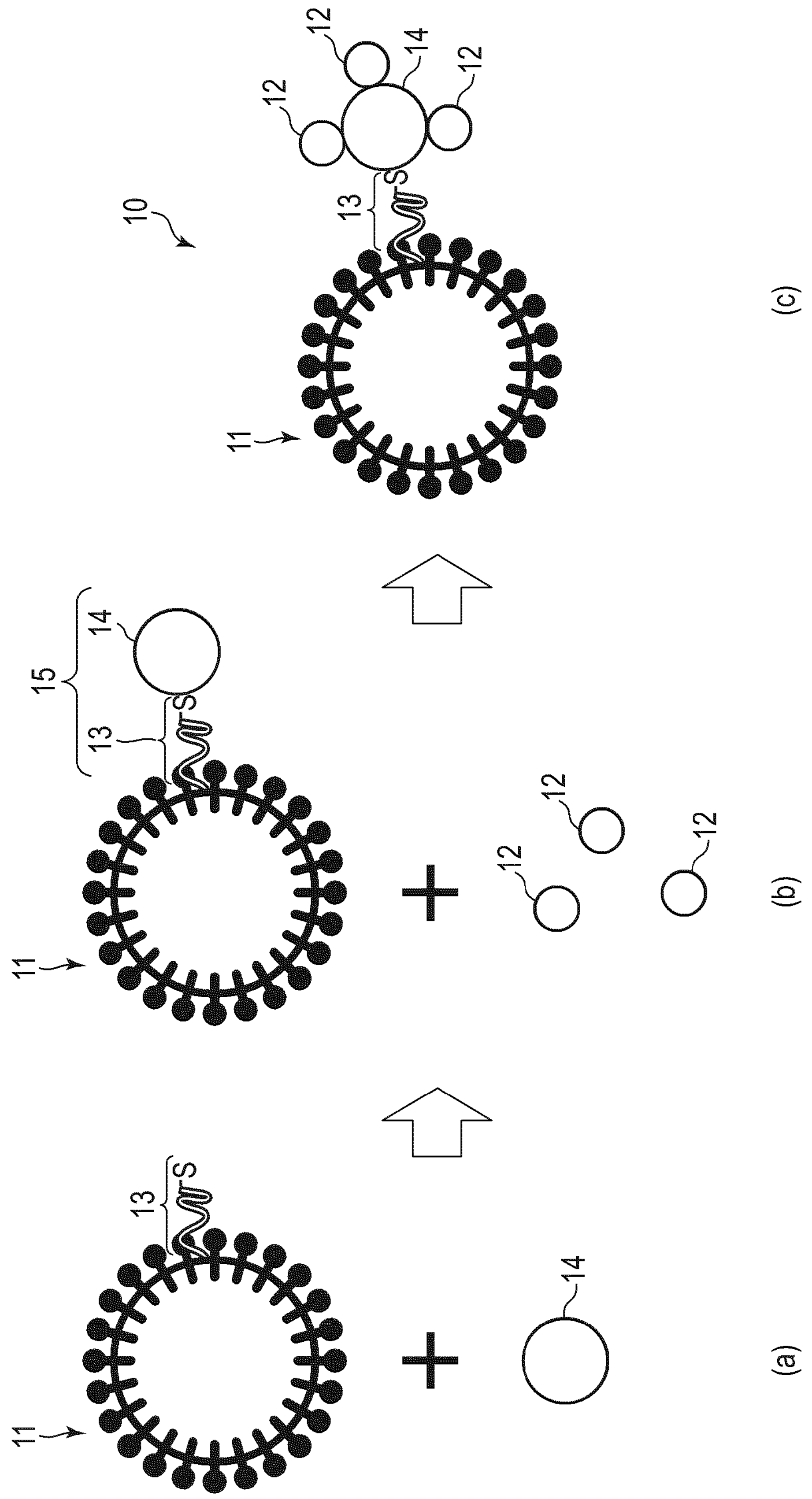


FIG. 2

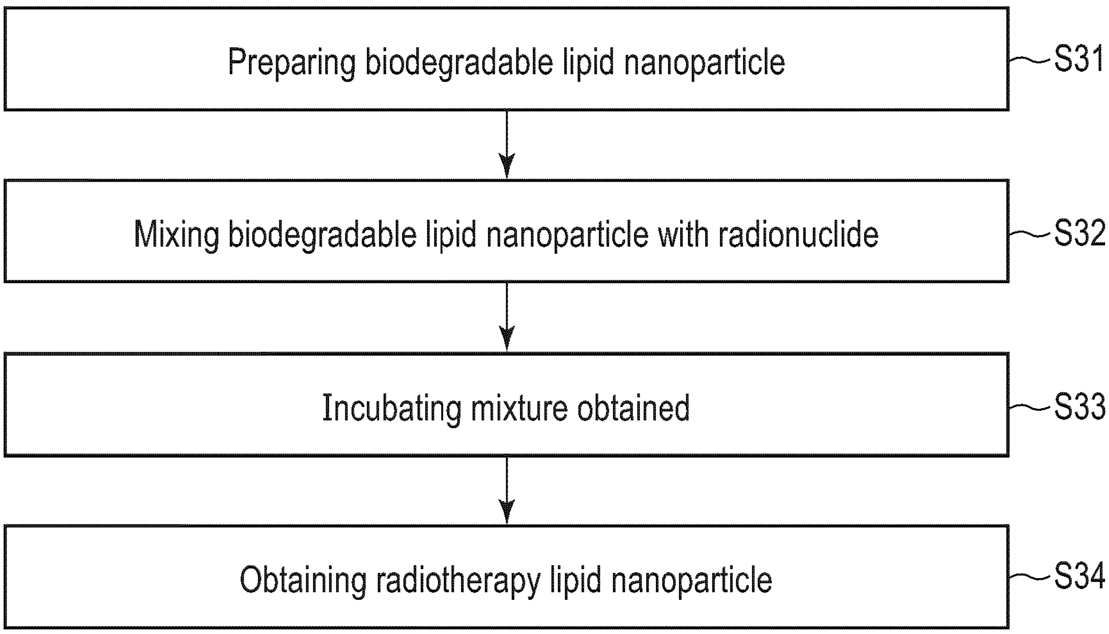
Preparing biodegradable lipid nanoparticle
S31
Mixing biodegradable lipid nanoparticle with radionuclide
S32
Incubating mixture obtained
S33
Obtaining radiotherapy lipid nanoparticle
S34

F I G. 3

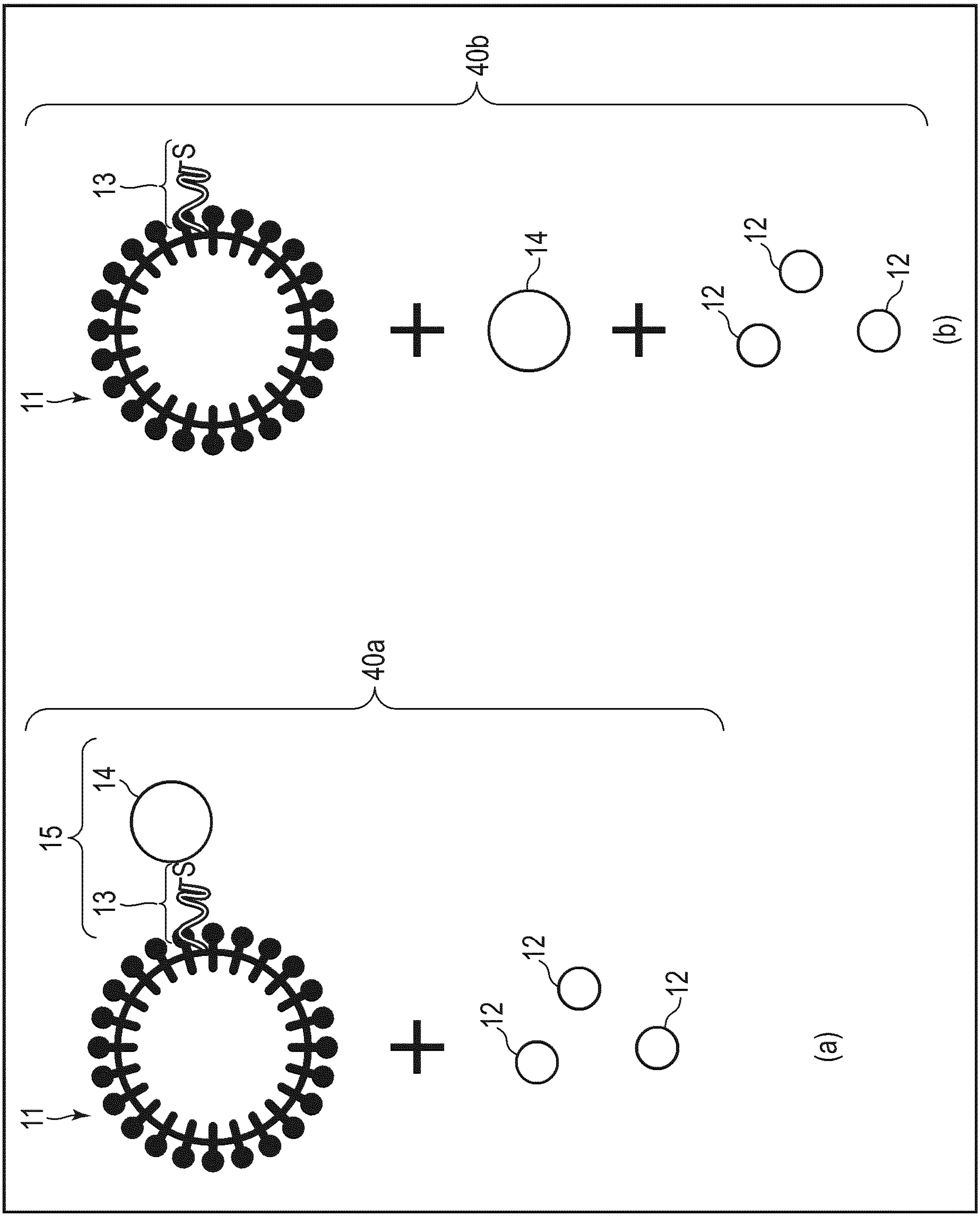


FIG. 4

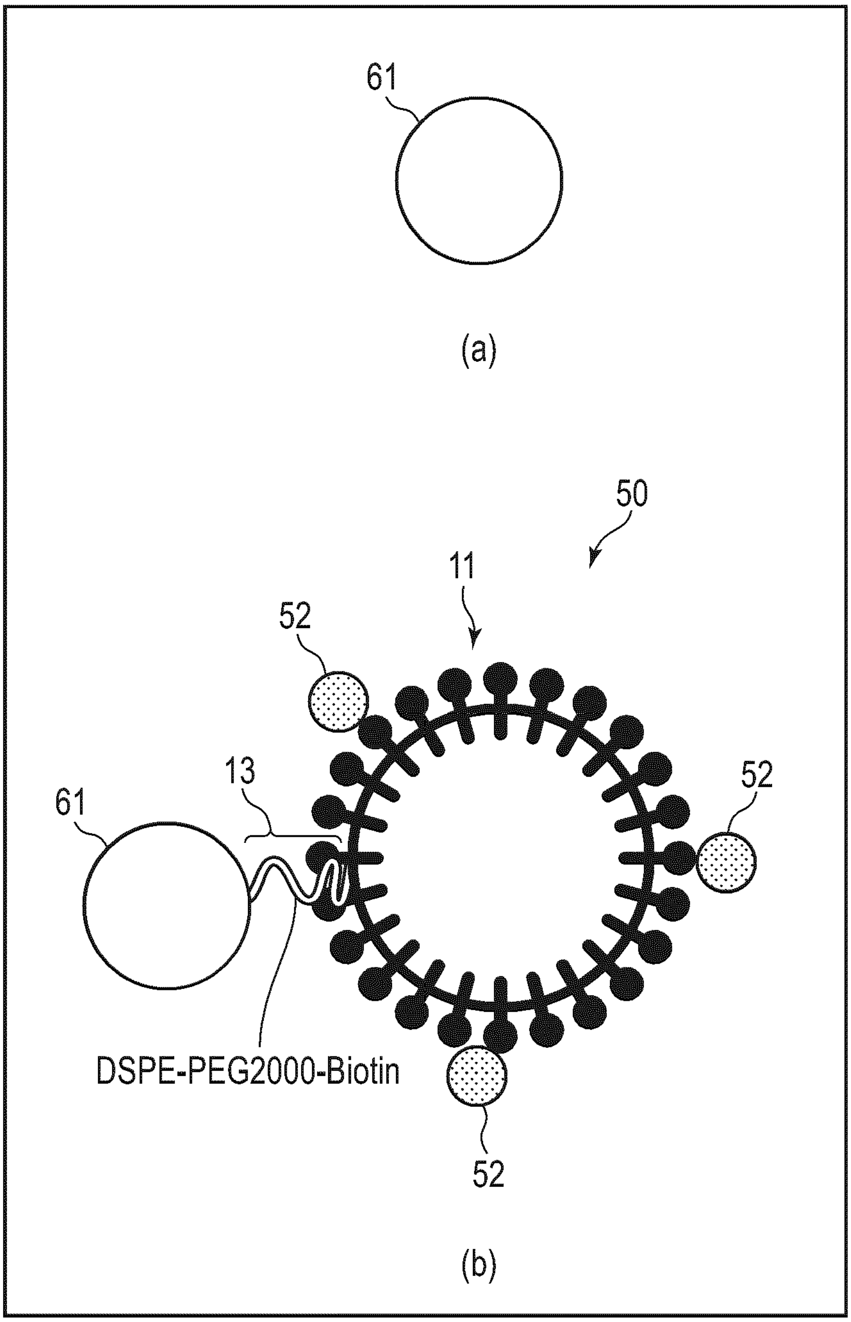


FIG. 5

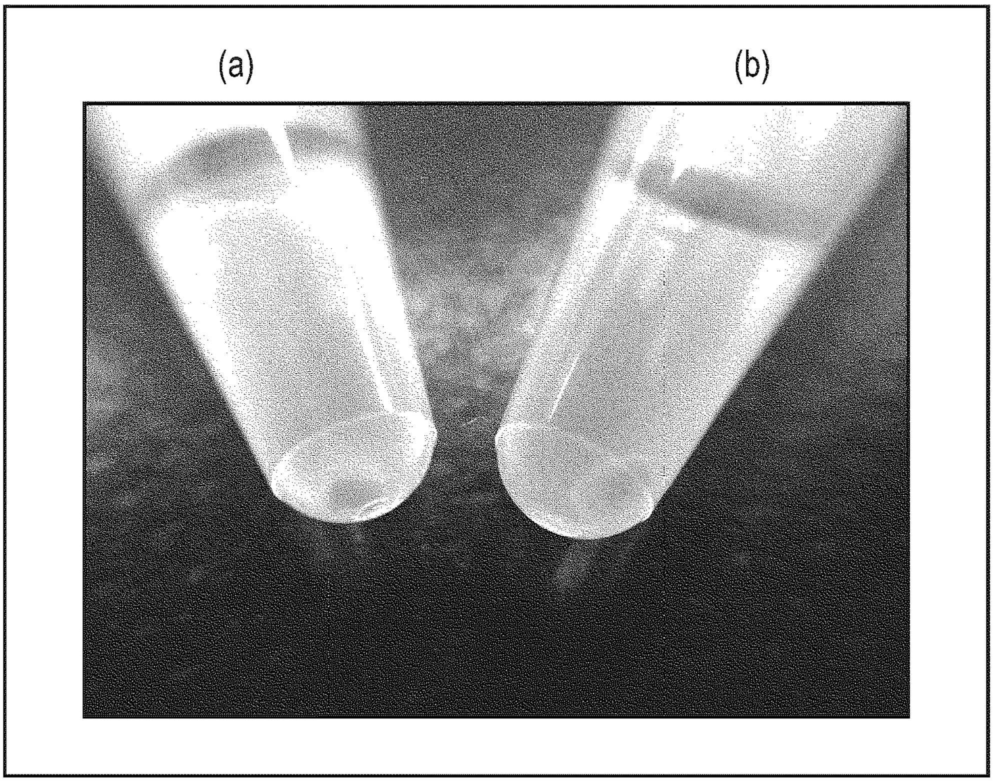


FIG. 6

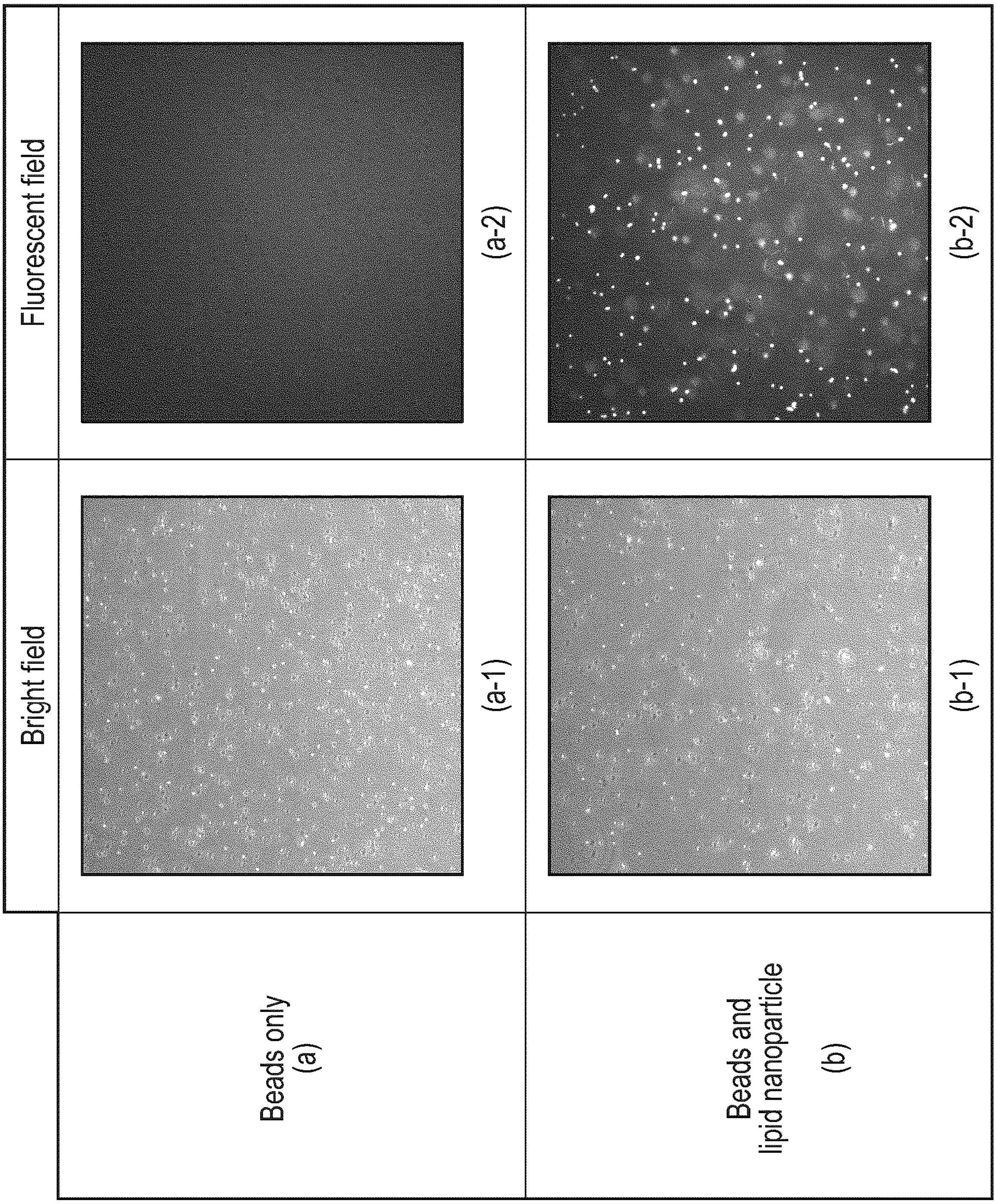
Bright field
Fluorescent field
Beads only
(a)
(a-1)
(a-2)
Beads and
lipid nanoparticle
(b)
(b-1)
(b-2)

F I G. 7

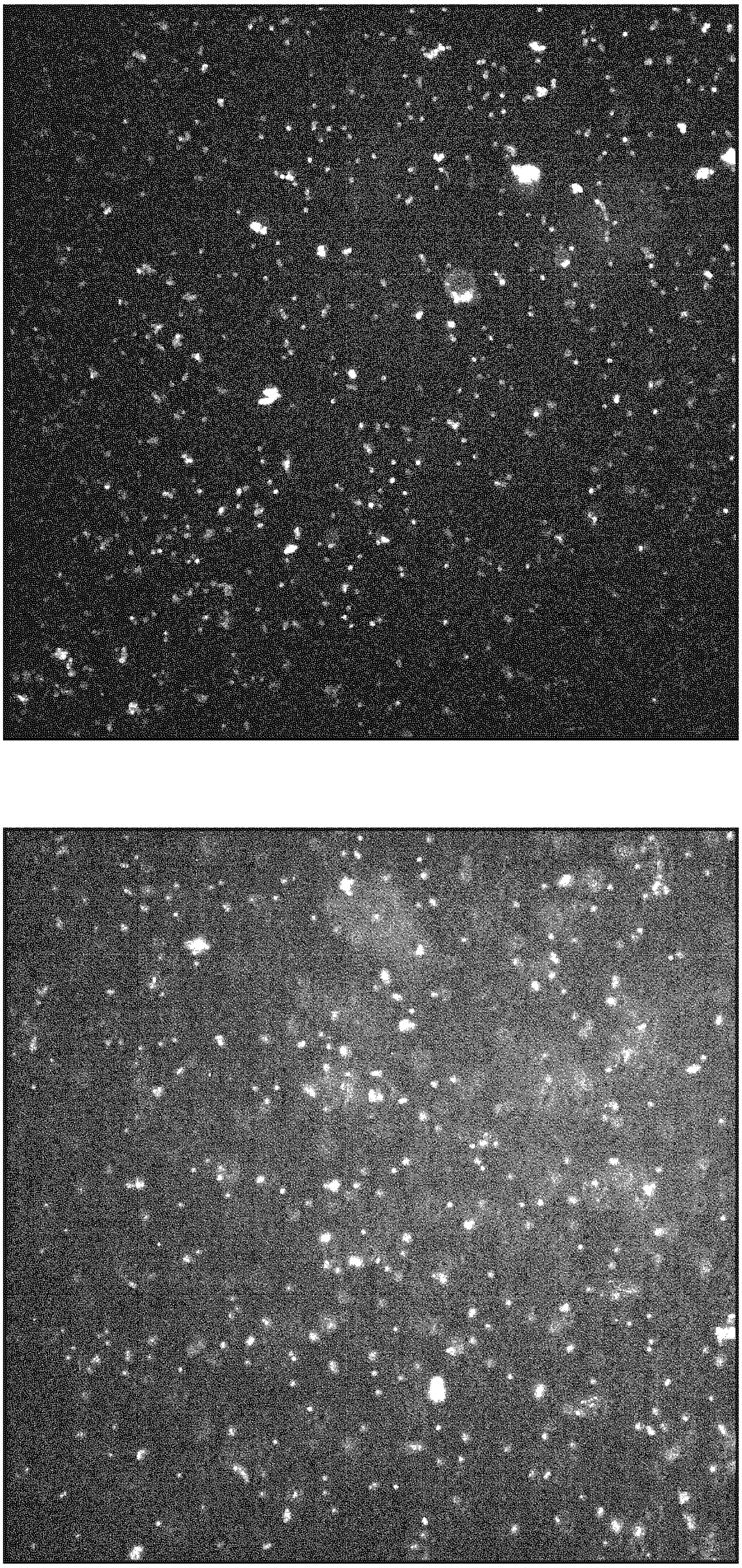

(a)

(b)

FIG. 8

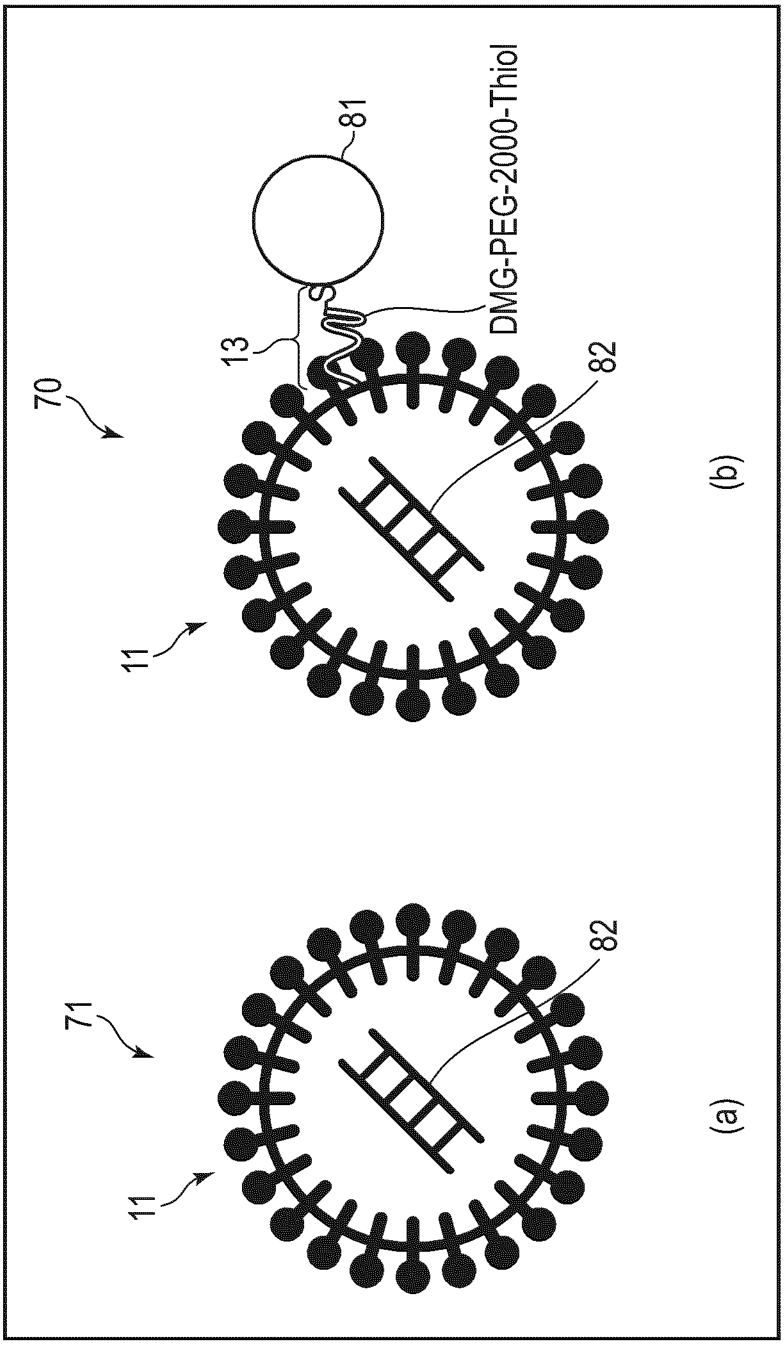


F I G. 9

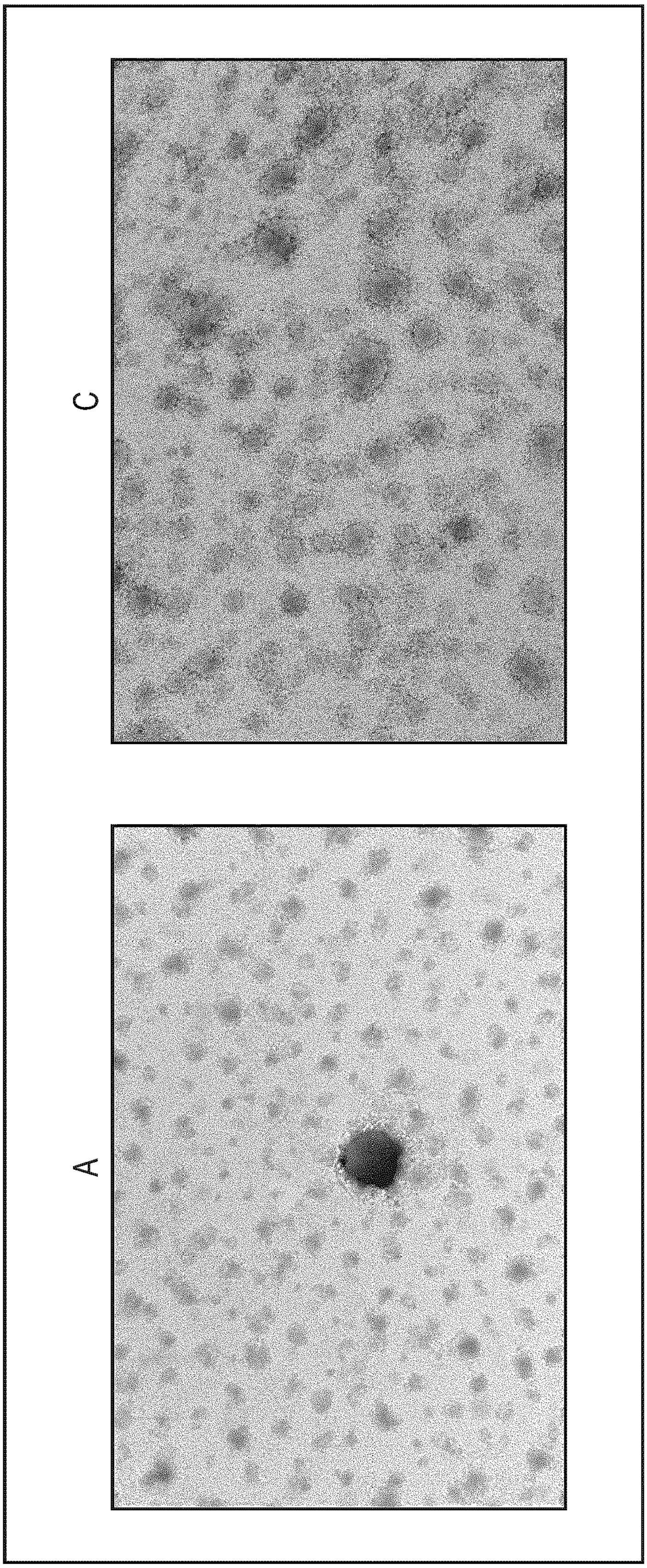


FIG. 10

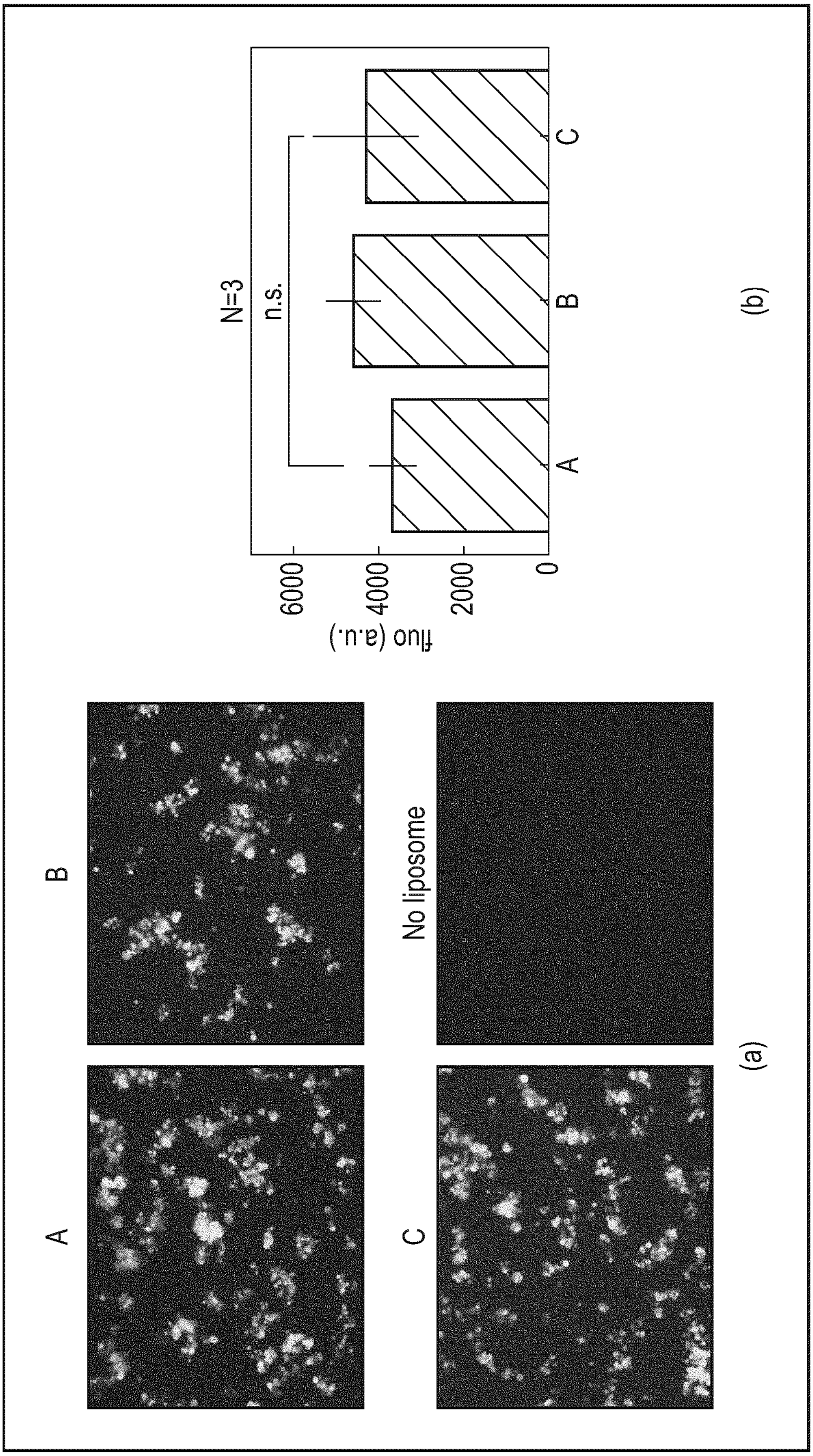


FIG. 11

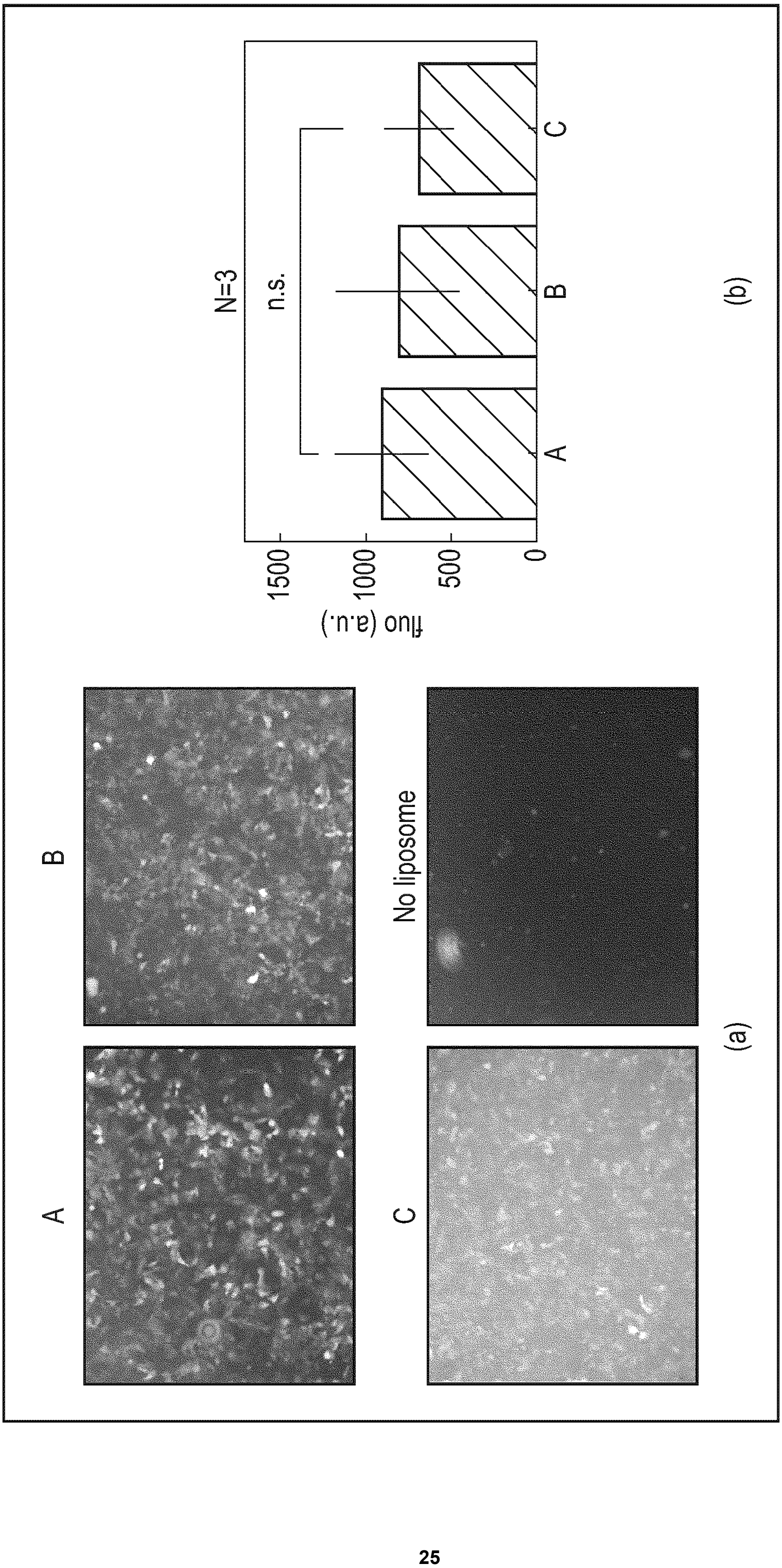


F I G. 12

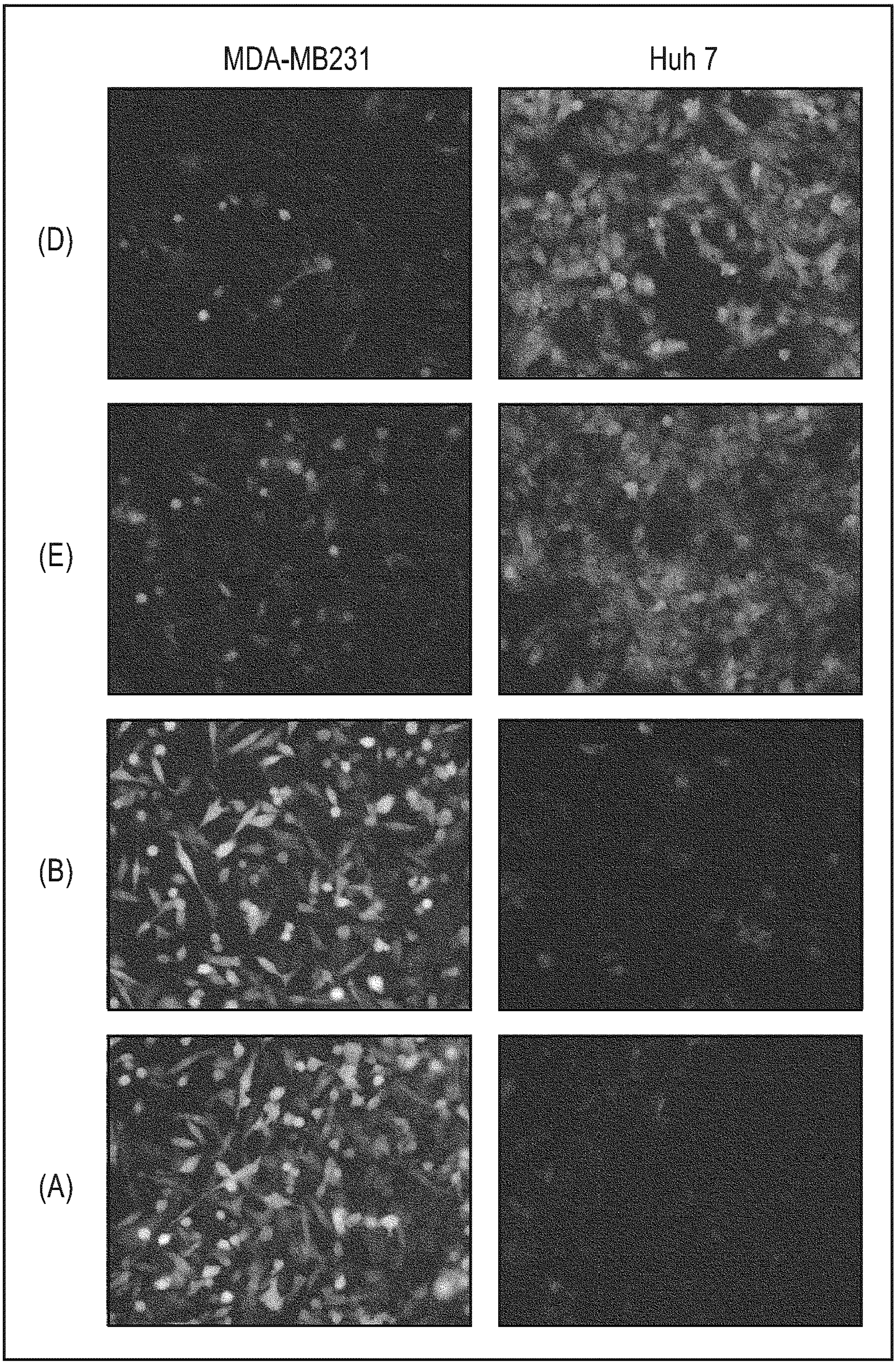


F I G. 13

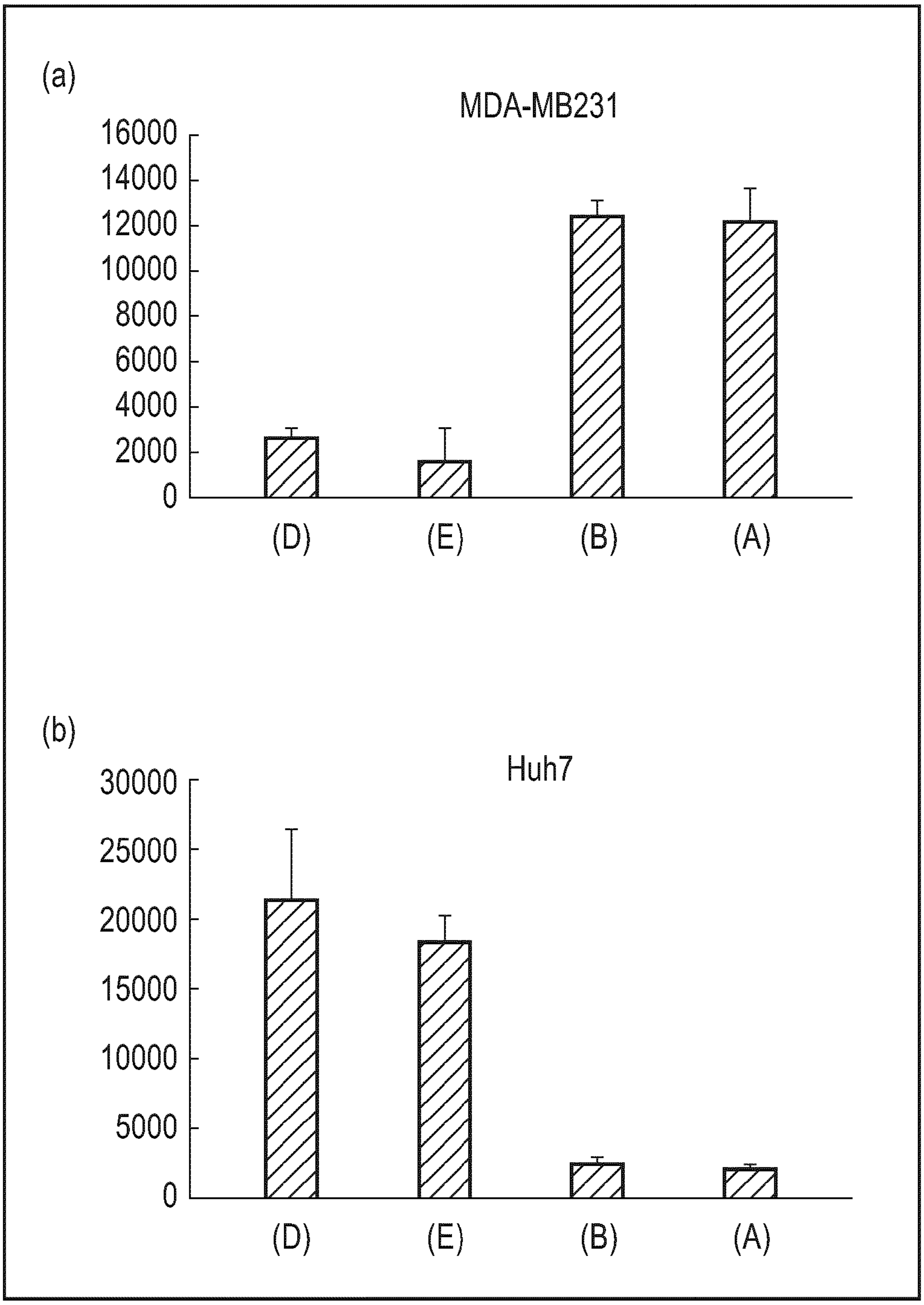
(a)
MDA-MB231
16000
14000
12000
10000
8000
6000
4000
2000
0
(D)
(E)
(B)
(A)
(b)
Huh7
30000
25000
20000
15000
10000
5000
0
(D)
(E)
(B)
(A)

F I G. 14

Europäisches Patentamt
European Patent Office
Office européen des brevets

## EUROPEAN SEARCH REPORT

Application Number
EP 25 15 3528

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 107 224 591 A (INST BIOPHYSICS CAS; UNIV BEIJING) 3 October 2017 (2017-10-03)<br>* claims 1, 2; figures 1, 2 *<br>----- | 1-4, 12-14 | INV.<br>A61K51/12<br>A61K9/51<br>A61P35/00 |
| X | SATTIRAJU ANIRUDH ET AL: "Alpha Particle Enhanced Blood Brain/Tumor Barrier Permeabilization in Glioblastomas Using Integrin Alpha-v Beta-3-Targeted Liposomes",<br>MOLECULAR CANCER THERAPEUTICS,<br>vol. 16, no. 10,<br>1 October 2017 (2017-10-01), pages 2191-2200, XP093286419,<br>US<br>ISSN: 1535-7163, DOI: 10.1158/1535-7163.MCT-16-0907<br>Retrieved from the Internet:<br>URL:https://watermark.silverchair.com/2191.pdf?token=AQECAHi208BE49Ooan9kkhW_Ercy7Dm3ZL_9Cf3qfKAc485ysgAAA0kwggNFBgkqhkiG9w0BBwagggM2MIIDMgIBADCCAysGCSqGSIb3DQEHATAeBglghkgBZQMEAS4wEQQMMtctMAuanHm-wPZkAgEQgIIC_Ave62utSwRSPVPTPOfGnaSdl0ebFwCAaSGPWIA8bo-Sg5qoo5BfXlUxN715rfltlHLoPADHhFXPWN57zeEGgoz6Sk68r4s><br>* abstract *<br>* figure 1 *<br>----- | 1-3, 11-14 | |

-/--

TECHNICAL FIELDS SEARCHED (IPC)

A61K

The present search report has been drawn up for all claims

| Place of search | Date of completion of the search | Examiner |
|---|---|---|
| The Hague | 16 June 2025 | Langer, Miren |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

3
EPO FORM 1503 03.82 (P04C01)

Europäisches Patentamt
European Patent Office
Office européen des brevets

# EUROPEAN SEARCH REPORT

Application Number
EP 25 15 3528

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KLEYNHANS JANKE ET AL: "Obstacles and Recommendations for Clinical Translation of Nanoparticle System-Based Targeted Alpha-Particle Therapy", MATERIALS, vol. 14, no. 17, 24 August 2021 (2021-08-24), page 4784, XP093286360, ISSN: 1996-1944, DOI: 10.3390/ma14174784 Retrieved from the Internet: URL:https://www.mdpi.com/1996-1944/14/17/4784/pdf> | 1-15 | |
| Y | * page 3 - page 5; table 1 * | 1-15 | |
| Y | KOGA KENTO ET AL: "Gold nanoparticle-coated thermosensitive liposomes for the triggered release of doxorubicin, and photothermal therapy using a near-infrared laser", COLLOIDS AND SURFACES A : PHYSIOCHEMICAL AND ENGINEERINGS ASPECTS, ELSEVIER, AMSTERDAM, NL, vol. 626, 18 June 2021 (2021-06-18), XP086735399, ISSN: 0927-7757, DOI: 10.1016/J.COLSURFA.2021.127038 [retrieved on 2021-06-18] * abstract * * page 2; figure 1 * | 1-15 | TECHNICAL FIELDS SEARCHED (IPC) |
| Y | WO 2021/186233 A1 (UNIV SHINSHU [JP]; TOSHIBA KK [JP]) 23 September 2021 (2021-09-23) * example 1 * * figures 8, 9, 10 * | 1-15 | |
| | -/-- | | |

The present search report has been drawn up for all claims

| Place of search | Date of completion of the search | Examiner |
|---|---|---|
| The Hague | 16 June 2025 | Langer, Miren |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 25 15 3528

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | MA RUNPU ET AL: "Lipid nanoparticles: A delicate nucleic acid delivery system to be further explored", NANO TODAY, ELSEVIER, AMSTERDAM, NL, vol. 61, 7 December 2024 (2024-12-07), XP087715727, ISSN: 1748-0132, DOI: 10.1016/J.NANTOD.2024.102586 [retrieved on 2024-12-07] ----- | 1-15 | |

TECHNICAL FIELDS SEARCHED (IPC)

The present search report has been drawn up for all claims

3

| Place of search | Date of completion of the search | Examiner |
|---|---|---|
| The Hague | 16 June 2025 | Langer, Miren |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 25 15 3528

This annex lists the patent family members relating to the patent documents cited in the above-mentioned European search report. The members are as contained in the European Patent Office EDP file on
The European Patent Office is in no way liable for these particulars which are merely given for the purpose of information.

16-06-2025

| Patent document cited in search report | Publication date | Patent family member(s) | Publication date |
|---|---|---|---|
| CN 107224591 A | 03-10-2017 | NONE | |
| WO 2021186233 A1 | 23-09-2021 | CN 113710233 A | 26-11-2021 |
| | | EP 4120999 A1 | 25-01-2023 |
| | | JP 7244034 B2 | 22-03-2023 |
| | | JP 2021147353 A | 27-09-2021 |
| | | JP 2022529765 A | 24-06-2022 |
| | | US 2022047517 A1 | 17-02-2022 |
| | | WO 2021186233 A1 | 23-09-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6320017 B


### Non-patent literature cited in the description


- On AunAt clusters as potential astatine carriers. *RCS Advances*, 2017
- **SHIGEKI WATANABE et al.** Separation of At-211 by Dry and Wet Methods. *15th Annual Meeting on Radiopharmaceuticals and Imaging Agents*, 2015